# EUROPEAN PATENT APPLICATION

(11) **EP 4 246 528 A1**
(43) Date of publication of application: **20.09.2023**
(21) Application number: 23161898.4
(22) Date of filing: 14.03.2023
(51) Int. Cl.: G16H 50/20, G16H 50/30, G16H 20/70

(54) **ADAPTIVE AND CONFIGURABLE DELIVERY OF MEASUREMENT BASED CARE TO ASSESS BEHAVIORAL HEALTH STATUS**

(30) Priority: 14.03.2022 US 202263319437 P
(71) Applicant: Health Rhythms, Inc., New York, NY 11101 (US)
(72) Inventor: MATTHEWS, Mark, New York, 11101 (US); SIMONS, Tad, New York, 11101 (US); LARSON, Mckay Lyall, New York, 11101 (US); JADZINSKY, Pablo Daniel, New York, 11101 (US); LEACH, Jeremy, New York, 11101 (US); ARANOVICH, Gabriel, New York, 11101 (US)
(74) Representative: Morrall, Jonathan Ian McLachlan

(57) **Abstract**

In one embodiment, a computer-implemented method can receive, from a mobile computing device, a measurement-based care (MBC) assessment request from a patient. The method can asynchronously assess, using the application server, a plurality of MBC assessment data from the patient, wherein the plurality of MBC assessment data comprises passively collected data values, assessments, programmed heuristics, programmed rules, and model thresholds. The method can asynchronously generate, using a machine learning model and the application server, a plurality of vital sign values for the patient using the plurality of MBC assessment data from the patient. The method can asynchronously determine, using a machine learning model and the application server, stratification group data for the patient using the plurality of MBC assessment data from the patient. The method can send, to the mobile computing device, instructions for presenting a user interface comprising the stratification group data for the patient.

## Description

### PRIORITY

This application claims the benefit under 35 U.S.C. § 119(e) of provisional application 63/319,437, filed 14 March 2022.

### COPYRIGHT NOTICE

A portion of the disclosure of this patent document contains material that is subject to copyright protection. The copyright owner has no objection to the facsimile reproduction by anyone of the patent document or the patent disclosure, as it appears in the Patent and Trademark Office patent file or records, but otherwise reserves all copyright or rights whatsoever. ^{©} 2022 Health Rhythms, Inc.

### TECHNICAL FIELD

One technical field of the present disclosure is computer-implemented methods of scheduling the delivery or presentation of surveys, forms, or other digital information. Another technical field is computer-implemented methods of attenuation stratification classification using machine learning models. Another technical field is computer-implemented methods of measurement-based self-report assessments. Another technical field is the computer-implemented assessment of mental health conditions of individuals.

### BACKGROUND

The approaches described in this section are approaches that could be pursued, but not necessarily approaches that have been previously conceived or pursued. Therefore, unless otherwise indicated, it should not be assumed that any of the approaches described in this section qualify as prior art merely by virtue of their inclusion in this section.

Numerous clinical studies over many years have shown that measurement-based care (MBC) assessments can improve behavioral health outcomes. MBC assessments use validated assessment parameters to quantify patient statuses, such as the PHQ-2, PHQ-8, PHQ-9, GAD-7, Altman Mania Scale, and others. MBC assessments can take extra time to administer in an office setting, and some MBC assessments can be delivered as a digital survey. Despite digitization, there has been no method of delivering these assessments at the most effective or desired moment to support the clinical workflow. Thus, clinicians may receive assessments that are not current, or not delivered when symptoms indicate a need to assess a patient. The end result is less informed treatment, unguided by timely quantitative measurements.

Furthermore, a wide range of needs relating to assessments is known including timing, type of assessment, and patient health status. Past practices have not been able to coordinate the need for assessments with the most appropriate delivery and collection of assessments. Current practice misses numerous opportunities for better measurement, which research supports as best practice.

### SUMMARY

The appended claims may serve as a summary of the invention.

### BRIEF DESCRIPTION OF THE DRAWINGS

In the drawings:
FIG. 1 illustrates a distributed computer system using an attention stratification application in accordance with one or more embodiments.
FIG. 2 illustrates a flow chart of a method of determining stratification group data for a patient in accordance with one or more embodiments.
FIG. 3A and FIG. 3B illustrate examples of a user interface of data for a clinician in accordance with one or more embodiments.
FIG. 4 illustrates an example graphical user interface in the display device for an individual patient in accordance with one or more embodiments.
FIG. 5A illustrates an example graphical user interface that includes a landing page in accordance with one or more embodiments.
FIG. 5B illustrates an example graphical user interface for the display of personal data via the mobile app in accordance with one or more embodiments.
FIG. 5C illustrates an example graphical user interface that shows the consumption of alcoholic beverages by a user of a mobile computing device in accordance with one or more embodiments.
FIG. 5D illustrates an example graphical user interface that shows detailed data for weekly sleep periods in accordance with one or more embodiments.
FIG. 5E illustrates an example graphical user interface that shows detailed data for monthly sleep periods in accordance with one or more embodiments.
FIG. 5F illustrates an example graphical user interface that shows detailed data for monthly activity in accordance with one or more embodiments.
FIG. 6 illustrates an example graphical user interface that displays health scores of a user of a mobile computing device in accordance with one or more embodiments.
FIG. 7 illustrates an example assessment interface in accordance with one or more embodiments.
FIG. 8 illustrates a computer system in accordance with one or more embodiments.

### DETAILED DESCRIPTION

In the following description, for the purposes of explanation, numerous specific details are set forth in order to provide a thorough understanding of the present invention. It will be apparent, however, that the present invention may be practiced without these specific details. In other instances, well-known structures and devices are shown in block diagram form in order to avoid unnecessarily obscuring the present invention.

The text of this disclosure, in combination with the drawing figures, is intended to state in prose the algorithms that are necessary to program the computer to implement the claimed inventions, at the same level of detail that is used by people of skill in the arts to which this disclosure pertains to communicate with one another concerning functions to be programmed, inputs, transformations, outputs and other aspects of programming. That is, the level of detail set forth in this disclosure is the same level of detail that persons of skill in the art normally use to communicate with one another to express algorithms to be programmed or the structure and function of programs to implement the inventions claimed herein.

One or more different inventions may be described in this disclosure, with alternative embodiments to illustrate examples. Other embodiments may be utilized and structural, logical, software, electrical, and other changes may be made without departing from the scope of the particular inventions. Various modifications and alterations are possible and expected. Some features of one or more of the inventions may be described with reference to one or more particular embodiments or drawing figures, but such features are not limited to usage in the one or more particular embodiments or figures with reference to which they are described. Thus, the present disclosure is neither a literal description of all embodiments of one or more of the inventions nor a listing of features of one or more of the inventions that must be present in all embodiments.

Headings of sections and the title are provided for convenience but are not intended as limiting the disclosure in any way or as a basis for interpreting the claims. Devices that are described as in communication with each other need not be in continuous communication with each other, unless expressly specified otherwise. In addition, devices that are in communication with each other may communicate directly or indirectly through one or more intermediaries, logical or physical.

A description of an embodiment with several components in communication with one other does not imply that all such components are required. Optional components may be described to illustrate a variety of possible embodiments and to fully illustrate one or more aspects of the inventions. Similarly, although process steps, method steps, algorithms, or the like may be described in sequential order, such processes, methods, and algorithms may generally be configured to work in different orders, unless specifically stated to the contrary. Any sequence or order of steps described in this disclosure is not a required sequence or order. The steps of described processes may be performed in any order practical. Further, some steps may be performed simultaneously. The illustration of a process in a drawing does not exclude variations and modifications, does not imply that the process or any of its steps are necessary to one or more of the invention(s), and does not imply that the illustrated process is preferred. The steps may be described once per embodiment, but need not occur only once. Some steps may be omitted in some embodiments or some occurrences, or some steps may be executed more than once in a given embodiment or occurrence. When a single device or article is described, more than one device or article may be used in place of a single device or article. Where more than one device or article is described, a single device or article may be used in place of the more than one device or article.

The functionality or the features of a device may be alternatively embodied by one or more other devices that are not explicitly described as having such functionality or features. Thus, other embodiments of one or more of the inventions need not include the device itself. Techniques and mechanisms described or referenced herein will sometimes be described in singular form for clarity. However, it should be noted that particular embodiments include multiple iterations of a technique or multiple manifestations of a mechanism unless noted otherwise. Process descriptions or blocks in figures should be understood as representing modules, segments, or portions of code which include one or more executable instructions for implementing specific logical functions or steps in the process. Alternate implementations are included within the scope of embodiments of the present invention in which, for example, functions may be executed out of order from that shown or discussed, including substantially concurrently or in reverse order, depending on the functionality involved.

Embodiments are described in sections below according to the following outline:
1. GENERAL OVERVIEW
2. STRUCTURAL AND FUNCTIONAL OVERVIEW
   2.1 ATTENTION STRATIFICATION APPLICATION
   2.2 FUNCTIONAL OVERVIEW
3. PROCEDURAL OVERVIEW
4. IMPLEMENTATION EXAMPLES
   4.1 COLLECTING DATA FROM PATIENTS
   4.2 DELIVERING DATA TO CLINICIANS
5. HARDWARE OVERVIEW

### 1. GENERAL OVERVIEW

In an embodiment, a computer-implemented method can be programmed for optimal MBC assessments of the mental health conditions of a patent which are integrated into treatment or a study plan for the patent. The computer-implemented method can deliver the MBC assessments based on a plurality of triggering factors: 1) at regular intervals as in support of a clinical trial protocol for a CNS medication, 2) according to calendar events before/after an office visit, 3) according to clinician's request for additional patient information based on changes in patient behavior, 4) according to signals from patient monitoring using sensors that monitor sleep, activity, social engagement, and circadian rhythms, and derived inferences from such signals, and 5) according to rules about the history and timing of past assessments, such as too many, too few, or allowed billing cycles, etc. In particular, the computer-implemented method can be programmed to schedule the delivery or presentation of surveys, forms, or other digital information.

One technical benefit of an embodiment is to improve the user experience for clinicians to assess MBC measurements for the patient. In an embodiment, the computer-implemented method can include a computer-implemented and human-computer interface to receive data to configure MBC for specific purposes and patient needs, including for individuals or groups. In one embodiment, the computer-implemented method can include a plurality of configuration algorithms for various purposes. For example, the plurality of configuration algorithms can be programmed to allow the selection of an individual patient or specify a related group of patients. As another example, the plurality of configuration algorithms can be programmed to allow the selection of a predetermined list of assessments. As another example, the plurality of configuration algorithms can be programmed to automatically determine the best timing to deliver MBC assessments based on the selections previously entered and the factors described above. As another example, the plurality of configuration algorithms can be programmed to allow clinicians to override the selection and order an assessment. In an embodiment, the plurality of configuration algorithms may be programmed as applications that are embedded in electronic health records (EHR) or in automated survey engines.

One technical benefit of an embodiment is to improve the MBC assessments for a patient using one or more machine learning models. In an embodiment, the computer-implemented method can receive an MBC assessment request from a patient. In response to the MBC assessment request from the patient, the computer-implemented method can asynchronously assess a plurality of MBC assessment data from the patient. For example, the plurality of MBC assessment data comprises passively collected data values, assessments, programmed heuristics, programmed rules, and model thresholds. The computer-implemented method can use one or more machine learning models to determine a plurality of vital sign values for the patient using the plurality of MBC assessment data from the patient. For example, the computer-implemented method can use an autoencoder to determine the attention level of the patient using the plurality of MBC assessment data from the patient. The autoencoder includes a contrastive loss function based on a first distance between anchor data and positive data, a second distance between the anchor data and negative data, and a margin. As another example, the computer-implemented method can use a supervised machine learning model to determine stratification group data for multiple patients by ranking the patients based on their attention levels and assigning a category to each of the patients.

One technical benefit of an embodiment is to improve the MBC assessments based on stratification group data determined using one or more machine learning models. In an embodiment, the computer-implemented method can include an attention stratification application that is programmed to selectively submit a plurality of different queries or surveys to patients based upon a schedule or configuration that varies according to responses to preceding queries. By collecting responses to specified queries from the patient and conducting analytics on the data according to a specific programmed process, the application can deliver output to a clinician that enables an accurate assessment of the patient's state. For example, the attention stratification application can implement flexible configuration and scheduling of surveys, questionnaires, and other data collection functions. As another example, the attention stratification application is programmed to selectively call or use one or more functions, such as self-report trigger logic, anomaly detection trigger logic, an insights trigger, and a random trigger to determine one or more data collection actions to initiate in relation to different users based on different datasets that have been collected from the different users over time.

In an embodiment, attention stratification logic 126 comprises four elements of trigger logic denoted self-report trigger logic 118, anomaly detection trigger logic 120, insights trigger 122, and random trigger 124. One or more of the triggers or trigger logic can be based on a machine learning classifier 136 and an autoencoder 138. Rather than providing a heuristic-based system for predicting attention levels, embodiments use machine learning for predicting attention levels. One or more embodiments can be programmed to predict attention levels with labels for classification, with no encoder; for example, embodiments can use an ANN classifier, such as gradient boost, decision trees, random forest, or metric learning. One or more embodiments can use an encoder 138, like a contrastive classifier. Other embodiments can use no labels for prediction.

### 2. STRUCTURAL AND FUNCTIONAL OVERVIEW

### 2.1 ATTENTION STRATIFICATION APPLICATION

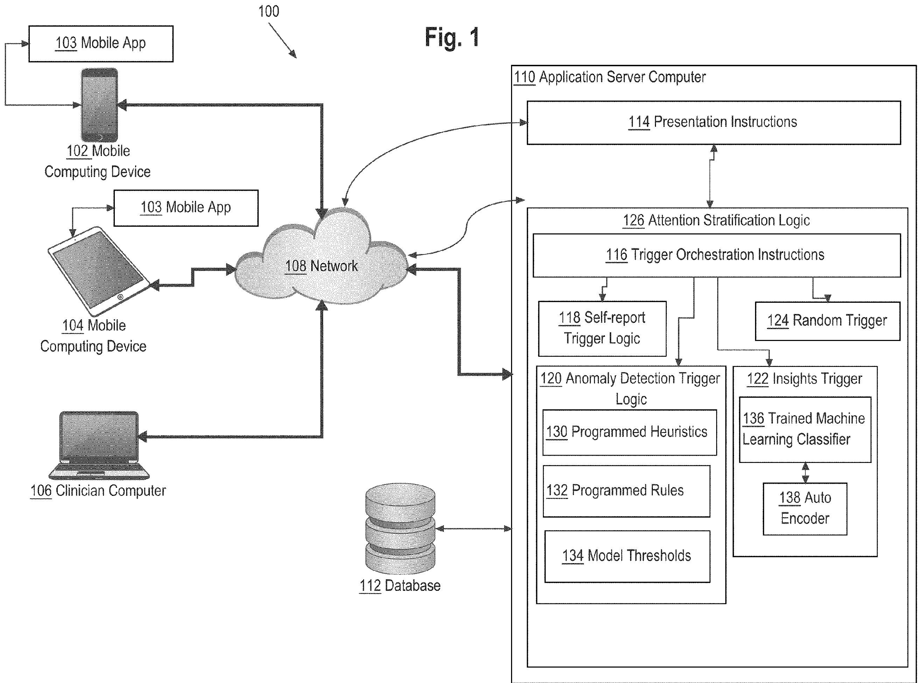

FIG. 1 illustrates a distributed computer system using an attention stratification application in accordance with one or more embodiments. In particular, FIG. 1 shows a distributed computer system showing the context of use and principal functional elements with which one embodiment could be implemented. In an embodiment, a computer system 100 comprises components that are implemented at least partially by hardware at one or more computing devices, such as one or more hardware processors executing stored program instructions stored in one or more memories for performing the functions that are described herein. In other words, all functions described herein are intended to indicate operations that are performed using programming in a special-purpose computer or general-purpose computer, in various embodiments. FIG. 1 illustrates only one of many possible arrangements of components configured to execute the programming described herein. Other arrangements may include fewer or different components, and the division of work between the components may vary depending on the arrangement.

FIG. 1, and the other drawing figures and all of the description and claims in this disclosure, are intended to present, disclose and claim a technical system and technical methods in which specially programmed computers, using a special-purpose distributed computer system design, execute functions that have not been available before to provide a practical application of computing technology to the problem of machine learning model development, validation, and deployment. In this manner, the disclosure presents a technical solution to a technical problem, and any interpretation of the disclosure or claims to cover any judicial exception to patent eligibility, such as an abstract idea, mental process, method of organizing human activity or mathematical algorithm, has no support in this disclosure and is erroneous.

In the example of FIG. 1, a distributed computer system 100 comprises mobile computing devices 102, 104 that are communicatively coupled via network 108 to an application server computer 110. Each of the mobile computing devices 102, 104 may comprise any kind of computing device such as a laptop computer, tablet computer, or smartphone. While the nature of the applications herein can benefit from the use of mobile computing devices, in some embodiments, a desktop computer or workstation could be used. For clarity, FIG. 1 shows two mobile computing devices 102, 104, but in practical embodiments, the system 100 can include thousands to millions of mobile computing devices depending upon the processing capacity of the application server computer 110.

For purposes of illustrating a clear example, the mobile computing devices 102, 104 are described as associated with system users, such as patients who are undergoing monitoring or evaluation for one or more health conditions, including but not limited to mental health conditions. A clinician computer 106 also is communicatively coupled via network 108 to an application server computer 110. The clinician computer 106 may comprise any kind of computing device such as a laptop computer, tablet computer, smartphone, desktop computer or workstation, and can be associated with a healthcare provider or practitioner who is one or more members of a care team for the patients who use the mobile computing devices 102, 104.

Each of the mobile computing devices 102, 104, and clinician computer 106 executes application programs including a browser, which can comprise any application program that is compatible with open protocols such as HTTP and HTML; commercially available examples include CHROME, SAFARI, and EDGE. In an embodiment, mobile computing devices 102, 104, and clinician computer 106 interact with application server computer 110 using the browser to transmit requests for network resources and to receive and render the network resources in the browser using a display device. Examples of network resources can be dynamically generated web pages or HTML code; other sections of this description show examples of web pages with graphical user interfaces that can be rendered as the mobile computing devices 102, 104, and clinician computer 106 interoperate with the application server computer 110.

In some embodiments, mobile computing devices 102, 104 each execute a copy of a special-purpose application, denoted mobile app 103, that is programmed to implement the patient-side or user-side functions and screen displays that are described in other sections herein. In an embodiment, the mobile computing devices 102, 104 can interoperate with a compatible application or function elements hosted at the application server computer 110 using an application-specific protocol transported over HTTP or other transport protocols; this approach may enable simpler access to mobile computing device sensors, hardware elements, and system services, as well as in-app notifications. Or, mobile computing devices 102, 104, and clinician computer 106 can use only a browser and HTML for communication and presentation.

The application server computer 110 can access the mobile computing devices 102, 104, and clinician computer 106 through the network 108, which broadly represents any wireline or wireless network, using any of satellite or terrestrial network links, such as local area networks (LANs), metropolitan area networks (MANs), wide area networks (WANs), campus network, internetworks, or combinations thereof. The network 108 may include or comprise the public internet and networked server computers that implement Web2 and/or Web3 technologies. The network 108 may comprise or support intranets, extranets, or virtual private networks (VPNs). The distributed computer system 100 may be standalone or it may be part of a subsystem, which may, in turn, be part of a larger system, such as a legacy system. Thus, network 108 can provide digital electronic telecommunication between a visitor computer, such as mobile computing device 102, and the server computer, such as application server computer 110, using open protocols such as IP, TCP, HTTP. Likewise, network 108 can provide digital electronic telecommunication between an owner computer, such as clinician computer 106, and the application server computer 110, using open protocols such as IP, TCP, HTTP.

In an embodiment, application server computer 110 represents any computing device that is capable of responding to requests from and providing services to a large number of end station devices such as mobile computing devices 102, 104, and clinician computer 106. In various embodiments, application server computer 110 can comprise any of a single-machine processor, multi-processor machine, a processor or machine cluster, and/or one or more virtual computing instances in any of public data centers and private data centers. Application server computer 110 can be associated with a service provider, healthcare organization, government entity, or any other person or entity that mobile computing devices 102, 104, and clinician computer 106 could need to visit or interact with.

In an embodiment, application server computer 110 can execute an HTTP server that can respond to requests of the browsers of mobile computing devices 102, 104, and clinician computer 106 and can include a firewall, load balancer, or other infrastructure to manage large numbers of requests and responses. The application server computer 110 is coupled to a database 112, which can be implemented as a relational database, no-SQL database, or object store. Database 112 is configured using a table schema that can store, under security controls, data collected from mobile computing devices 102, 104, and transformed, formatted, or prepared for clinician computer 106 based on execution of the functional elements of the attention stratification logic 126.

### 2.2 FUNCTIONAL OVERVIEW

In an embodiment, application server computer 110 hosts or executes a web-based application which can be structured, in one embodiment, as a set of presentation instructions 114 and attention stratification logic 126. In an embodiment, the attention stratification logic 126 comprises trigger orchestration instructions 116 coupled to self-report trigger logic 118, anomaly detection trigger logic 120, an insights trigger 122, and a random trigger 124. In particular, the presentation instructions 114 are programmed to generate dynamic HTML or application-specific presentation instructions to transmit via network 108 to the mobile computing devices 102, 104, and clinician computer 106, in response to calls, signals, or requests from other function elements of the application server computer 110. For example, presentation instructions 114 can be programmed to transform calls from other elements of the application server computer 110 into app-compatible requests, posts, responses, or calls.

In an embodiment, the attention stratification logic 126 implements computer-implemented methods of grouping patients into strata, groups, or categories based upon data responses and analytics that are consistent with clinical concerns relevant to one or more healthcare diagnoses. In an embodiment, the trigger orchestration instructions 116 implement flexible configuration and scheduling of surveys, questionnaires, and other data collection functions and are programmed to selectively call or use one or more of self-report trigger logic 118, anomaly detection trigger logic 120, insights trigger 122, and random trigger 124. Each of the self-report trigger logic 118, anomaly detection trigger logic 120, an insights trigger 122, and a random trigger 124 can be programmed to determine, in a different way, when and what data collection actions to initiate in relation to different users, and based upon different datasets that have been collected from the different users over time.

In an embodiment, the programming of each of the self-report trigger logic 118, anomaly detection trigger logic 120, insights trigger 122, and random trigger 124 can use different forms of internal logic or functional elements. In one approach, as shown for the anomaly detection trigger logic 120, each of the self-report trigger logic 118, anomaly detection trigger logic 120, insights trigger 122, and random trigger 124 can be programmed to execute one or more programmed heuristics 130, programmed rules 132, or model thresholds 134, based on data that has been collected passively from the mobile computing devices 102, 104, or actively submitted by users of the mobile computing devices, to determine whether a particular question, survey, or questionnaire should be delivered to the devices, and in what form. The attention stratification logic 126 can be programmed to use programmed heuristics 130, programmed rules 132, such as clinical hard-coded rules, and/or model thresholds 134 to filter patients into attention strata, groups, or categories. For example, the attention stratification logic 126 can be programmed to diagnose a patent with bipolar and/or depression by assessing a plurality of MBC measurements, such PHQ-9, GAD-7, and Altman Mania Scale, based on a plurality of levels of acuity, such as "emergent", "urgent", "medium", "no action", etc. As another example, the attention stratification logic 126 can include different assessment triggers for the MBC measurements. In particular, the assessment can be automatically triggered by an algorithm determined by a user, such as "Min frequency is 1 per month for assessments applicable to the patient" and/or "No max frequency assigned in MVP." Likewise, the assessment can be triggered by a provider, such as the user. An example of assessments and diagnoses for stratification can be found in Table 1 below.

**Table 1. An example of assessments and diagnoses for stratification.**

| Diagnoses: | Bipolar, Depression |
|---|---|
| Assessments: | PHQ-9, GAD-7, Altman Mania Scale |
| Levels of Acuity: | Emergent (red + active notification), Urgent (orange), Medium (yellow) and No Action. |
| Assessment triggers: | Algorithm triggered = Min frequency is 1 per month for assessments applicable to the patient. No max frequency assigned in MVP. |
| | Provider triggered = No min nor max frequency assigned. |

In an embodiment, the attention stratification logic 126 can diagnose a patent based on a plurality of metrics, such as programmed heuristics 130, programmed rules 132, and/or model thresholds 134. The attention stratification logic 126 can use programmed heuristics 130 to apply heuristics-based filtering to find similar patients. Programmed heuristics 130 can include empirical triggers and patient information without self-reports. For example, when a male patent is male has a low location entropy < 0.874, the patient has a medium level of acuity for the diagnoses of bipolar and depression. As another example, when a female patient has no children and a persistently low walking rate, the patient also has a medium level of acuity for the diagnoses of bipolar and depression. Programmed rules 132 can include clinical hard-coded rules, such as sensor-driven attention stratification and self-report. For example, when a patient has a PHQ-9 question 9 increase larger than 0, the patient has an emergent level of acuity for the diagnoses of bipolar and depression. As another example, when a patient has a PHQ-9 score in the range of 0-4, the patient has a low level of acuity for the diagnoses of bipolar and depression. Model thresholds 134 can include model triggers without self-reports. For example, when a patient has a behavior insight score in the range of 75-100, the patient has a low level of acuity for the diagnoses of bipolar and depression. As another example, when a patient has a behavior insight score less than 25, the patient has a medium level of acuity for the diagnoses of bipolar and depression. An example of programmable rules for stratification can be found in Table 2 below.

**Table 2. An example of programmed rules for stratification.**

| Metric | Criteria | Attention Stratification | Attention Indicator |
|---|---|---|---|
| Self-Report & Sensor Driven Attention Stratification | | | |
| PHQ-9 question 9 | response >0 | Urgent (3) | PHQ Question 9 score X indicates thoughts of self harm |
| PHQ-9 question 9 increase | score increase >0 | Emergent (4) | PHQ Question 9 score X indicates thoughts of self harm |
| PHQ-9 overall score | score ranges from 0-4 | Low (1) | PHQ X - minimal depression |
| PHQ-9 overall score | score ranges from 5-14 | Medium (2) | PHQ X - mild to moderate depression |
| PHQ-9 overall score | score ranges from 15-19 | Medium (2) | PHQ X - moderately severe depression |
| PHQ-9 overall score | score ranges from 20-27 when previous score was not in that range | Urgent (3) | PHQ X - severe depression (increase from score of X on date) or PHQ X - severe depression (no recent PHQ available) |
| PHQ-9 overall score | score ranges from 20-27 when previous score was also in that range | Medium (2) | PHQ X - Continued severe depression |
| Altman score | Question #3, score = 3, 4 | Urgent (3) | Altman Question 3 score X - indicating needs less sleep |
| Altman score | Question #3 score CHANGES to 4 | Emergent (4) | Altman Question 3 score increase to 4 - indicating does not need sleep |
| Altman score | score ranges from 0-5 | Low (1) | Altman - low indication of mania |
| Altman score | score ranges from 6 - 18 | Urgent (3) | Altman X - high probability of mania or hypomania |
| Altman score | score ranges from 18-20 | Emergent (4) | Altman X - very high probably of mania or hypomania |
| GAD-7 score | score ranges from 0-4 | Low (1) | GAD X - minimal anxiety |
| GAD-7 score | score ranges from 5-9 | Medium (2) | GAD X - mild anxiety |
| GAD-7 score | score ranges from 10-14 | Medium (2) | GAD X - moderate anxiety |
| GAD-7 score | score ranges from 15-21 when previous score was not in that range | Urgent (3) | |
| GAD-7 score | score ranges from 15-21 when previous score was also in that range | Medium (2) | |
| Bipolar diagnosis + sleep decrease > _ | 40% [last sleep duration vs previous week's median] | Urgent (3) | Patient's sleep has decreased 40% or more over previous week's median. |
| Bipolar diagnosis + sleep decrease > _ | No sleep in 2 days [DS: <3.0 hours, roughly bottom 1% of bipolar patients in RCT] | Urgent (3) | Patient has had minimal to no sleep in 2 days. |

| Empirical Triggers & Patient Info with no self-reports | | | |
|---|---|---|---|
| Male, low location entropy (risk multiple = 3.19x) | sex == 'Male' & loc_entropy < .874 | Medium (2) | Passive sensing of behavior patterns |
| Female with children, late wake time (risk multiple = 2.24x) | sex == 'Female' & children == 1 & sleep_end > 60900 | Medium (2) | Passive sensing of behavior patterns |
| Female without children, substantial increase in time at home (risk multiple = 2.03x) | sex == 'Female' & children == 0 & time_at_home_diff < - 35807 (s) | Medium (2) | Passive sensing of behavior patterns |
| Male, high avg time at home over past 7 days (risk multiple = 2.19x) | sex == 'Male' & time_at_home_m7 > 61776 (s) | Medium (2) | Passive sensing of behavior patterns |
| Female with children, persistently high chronotype score (risk multiple = 0.98x) | sex == 'Female' & children == 1 & chronotype_score_m7 > 3.014 | Medium (2) | Passive sensing of behavior patterns |
| Female without children, extremely high sustained activity events (risk multiple = 3.30x) | sex == 'Female' & children == 0 & ec_Activity_m7 > 1117 | Medium (2) | Passive sensing of behavior patterns |
| Diagnosed with Bipolar, persistently low walking rate (risk multiple = 5.88x) | bipolar == 1 & walking_rate_m7 < 1.316 | Medium (2) | Passive sensing of behavior patterns |
| Diagnosed with MDD, persistently long time at home (risk multiple = 0.80x) | mdd == 1 *&* time_at_home_m7 > 62046 | Medium (2) | Passive sensing of behavior patterns |
| Male, extremely high sustained variance in activity events (risk multiple = 1.86x) | sex == 'Male' & ec_Activity_diff_s14 > 259 | Medium (2) | Passive sensing of behavior patterns |
| Female with children, persistently high chronotype score (risk multiple = 0.69x) | sex == 'Female' & children == 1 & chronotype_score_m14 > 2.892 | Medium (2) | Passive sensing of behavior patterns |
| Female without children, persistently low walking rate (risk multiple = 1.34x) | sex == 'Female' & children == 0 & walking_rate_m14 < 1.106 | Medium (2) | Passive sensing of behavior patterns |
| Diagnosed with GAD, persistently low location entropy (risk multiple = 1.38x) | gad == 1 & loc_entropy_m14 < .978 | Medium (2) | Passive sensing of behavior patterns |
| Female with children, step count significantly falling (rel. to baseline) (risk multiple = 3.52x) | sex == 'Female' & children == 1 *&* step_count_diff_t_7_21 < - .037 | Medium (2) | Passive sensing of behavior patterns |
| Female without children, significantly increasing time at location 2 (often work/school) (risk multiple = 0.85x) | sex == 'Female' & children == 0 & time_at_location_2_diff_t_7_21 > .038 | Medium (2) | Passive sensing of behavior patterns |

| Model Triggers with no self-reports | | | |
|---|---|---|---|
| Insight Model Scores w/no self report within X | Behavior Insight Score <= 25 | Medium (2) | Passive sensing indicates high risk of depression |
| Insight Model Scores w/no self report within X | Behavior Insight Score 25 < score <= 50 | Medium (2) | Passive sensing indicates risk of depression |
| Insight Model Scores w/no self report within X | Behavior Insight Score 50 < score <= 75 | Low (1) | Passive sensing indicates low risk of depression |
| Insight Model Scores w/no self report within X | Behavior Insight Score 75 < score <= 100 | Low (1) | Passive sensing indicates low risk of depression |

| Miscellaneous | | | |
|---|---|---|---|
| No flag | If a patient is currently classed 'No Action' (aka "Low") and is not flagged OR a patient is flagged to complete a self-report and completes a self-report and is ok then class as 'No Action' | Low (1) | |

In an embodiment, any of the triggers or trigger logic, such as the self-report trigger logic 118, the anomaly detection trigger logic 120, the insights trigger 122, and the random trigger 124, can use one or more machine learning models, such as a trained machine learning classifier 136 and an auto encoder 138, to classify features associated with attention levels. The attention stratification logic 126 can apply the one or more machine learning models to rank patients by need of clinical attention using programmed heuristics 130, programmed rules 132, and model thresholds 134. For example, the trained machine learning classifier 136 is generated using a training dataset comprising millions of patient records representing the following features: inferred data values based on user activity, semantic location, passive social determinants of health, self-reported information on mental health state (i.e. labels for depression states, labels for mood states), information about a patient's past diagnosis, patient demographic details, clinical action labels indicating what action a clinician took on a presenting patient taken from an HER. An example of inferred data elements can be found in Table 3 below.

**Table 3. An example of inferred data elements**

| Inferred Data Metric | Example Meaning |
|---|---|
| hrinferences.infs.activity_percent | Percentage of time in which the user was active |
| hrinferences.infs.activity_stats_inf | Inferred magnitude of time in which the user was active |
| hrinferences.infs.app_suspension_inf | Inferred magnitude of time in which the user had suspended using the mobile application |
| hrinferences.infs.app_upgrade | A time at which the user upgraded the mobile application |
| hrinferences.infs.auth_inf | A time at which the user completed user authentication |
| hrinferences.infs.batches | |
| hrinferences.infs.chronotype | |
| hrinferences.infs.coverage | |
| hrinferences.infs.display_events | A number of events that have occurred on the mobile computing device that resulted in the display of information to the user |
| hrinferences.infs.gaps | |
| hrinferences.infs.home_location | A geographic location of the user's home based upon GPS data or similar device geo-location data |
| hrinferences.infs.home_location2 | A possible second geographic location of the user's home based upon GPS data or similar device geo-location data |
| hrinferences.infs.location_entropy | |
| hrinferences.infs.location_entropy2 | |
| hrinferences.infs.old_sleep | |
| hrinferences.infs.overlaps | |
| hrinferences.infs.peak_activity | |
| hrinferences.infs.radius_of_gyration | |
| hrinferences.infs.self_report | |
| hrinferences.infs.sleep_proxy | |
| hrinferences.infs.sleep_proxy_activity | |
| hrinferences.infs.sleep_proxy_display | |
| hrinferences.infs.sleep_proxy_pedometer | |
| hrinferences.infs.sleep_routine_index | |
| hrinferences.infs.step_rhythmicity | |
| hrinferences.infs.steps | A number of steps that the user took within a specified period |
| hrinferences.infs.steps_test_inf | |
| hrinferences.infs.time_at_home | An amount of time that the user spent at home |
| hrinferences.infs.time_leaving_home | The user's regular time of leaving home |
| hrinferences.infs.timezone_change | An indication that the user moved to a different time zone |
| hrinferences.infs.travel_diameter | A representation of an area in which the user recently traveled |
| hrinferences.infs.voice | A time at which the user placed a voice call with the mobile device |
| hrinferences.infs.walking_rate | The mean velocity of at which the user walks |
| hrinferences.infs.windowed_sleep_proxy | |
| hrinferences.infs.wsp2 | |

In an embodiment, the attention stratification logic 126 can asynchronously assessing a plurality of MBC assessment data from the patient which comprises passively collected data values, assessments, programmed heuristics, programmed rules, and model thresholds. The attention stratification logic 126 can use the trained machine learning classifier 136 to determine a plurality of vital sign values for the patient using the plurality of MBC assessment data from the patient. For example, the computer-implemented method can use an auto encoder to determine an attention level for the patient using the plurality of MBC assessment data from the patient. As another example, the computer-implemented method can use a supervised machine learning model, such as a decision tree model, to determine stratification group data for multiple patients by ranking the patients based on the attention levels and assigning a category to each of the patients. As another example, the computer-implemented method can use an unsupervised machine learning model, such as a k-means model, to determine natural clusters in the stratification group data for the multiple patients.

In an embodiment, the attention stratification logic 126 can apply a supervised machine learning algorithm, such as decision tree, random forest, support vector machine (SVM), to generate the trained machine learning classifier 136. In particular, the attention stratification logic 126 can select a plurality of attributes based on the features from the patient records. The attention stratification logic 126 can use a tree-like model to classify one or more natural clusters associated with the plurality of attributes into a map of possible outcomes, such as branches. For example, each internal node of the tree-like model represents a test on an attribute, each branch represents an outcome of the test, and each leaf node represents a class label. A path from root to leaf is determined based on a decision tree classification rule. In particular, a decision tree typically starts with a single node, which branches into possible outcomes. Each of those outcomes leads to additional nodes, which branch off into other possible outcomes. The accuracy of a decision tree model is controlled by a depth and a node splitting function of the decision tree model at the cost of increasing computation time. In an embodiment, the machine learning classifier 136 comprises a classification model having been trained using labels that have been collected from patients about their mental health states and from clinical actions that were taken, as represented in EHR records. The machine learning classifier 136 may be evaluated using one or more metrics, such as accuracy, sensitivity, specificity, precision, miss rate, false discovery rate, and false omission rate, using the measurements classified by the decision tree model.

Furthermore, the attention stratification logic 126 can apply an auto encoder 138 to determine an input to machine learning classifier 136. For example, the attention stratification logic 126 can apply the auto encoder 138 to determine a level of clinical attention for each patient. In an embodiment, the auto encoder 138 can include a contrastive loss function in accordance with one or more embodiments. The auto encoder 138 can include two neural networks based on convolutional neural networks (CNNs) with shared weights for a positive pair of anchor data and positive data and a negative pair of anchor data and negative data. The anchor data can include patient records of a first patient, such as a person with bipolar and depression. The positive data can include patent records of a second patient, such as a different person with bipolar and depression, who has similar symptoms or features in the patient records as the first patient. The negative data can include patient records of a third patient, such as a normal person, who has no similar symptoms or features in the patient records as the first patient. The CNN includes a plurality of hidden layers, such as a convolutional layer, a pooling layer, a rectified linear unit (ReLU) layer, a softmax layer, a regressor layer, a dropout layer, and/or various other hidden layer types. These hidden layers can be arranged in any order that satisfies the input/output size criteria. Each layer comprises of a set number of image filters. The output of filters from each layer is stacked together in the third dimension. This filter response stack then serves as the input to the next layer(s). The anchor data is convolved with a set of learned filters, designed to highlight specific characteristics of the input data set to determine anchor embedding, positive embedding, and negative embedding in a multidimensional embedding space. Furthermore, the contrastive loss function can be determined using the anchor embedding, positive embedding, and negative embedding based on positive pairs and negative pairs. In particular, the auto encoder 138 can calculate the contrastive loss function using a first distance between the anchor data and the positive data, a second distance between the anchor data and the negative data, and a margin. The auto encoder 138 can be determined by minimizing the first distance and maximizing the second distance until the difference between the first distance and the second distance is greater than the margin. As a result, the contrastive loss function can represent the difference between the similarity between the positive pairs and the difference between the similarity between the negative pairs.

Embodiments using machine learning classifier 136, with or without auto encoder 138, can avoid certain drawbacks of heuristics-based systems, such as maintenance and missing latent signals. Further, using auto encoder 138 a larger among of data becomes available to train the machine learning classifier 136, since the auto encoder is not trained against labels, but is trained to predict the set of inputs that will occur next in time. As in other machine learning domains, labels can be scarce while datasets can be large, and auto encoder and contrastive learning models of the kinds described herein provide ways of reducing the dimensionality of the data.

In an embodiment, the attention stratification logic 126 is programmed to continuously compute a plurality of vital sign values to clinicians automatically under stored program control. In an embodiment, for each of the plurality of vital sign values, the attention stratification logic 126 is programmed to generate a trend report associated with the corresponding vital based on input from passive device sensing, such as the passively collected data values, assessments, and programmed heuristics, and programmed rules. Trend reports may be programmed to communicate whether the particular vital is stable, going up or down over a period of time. A current state of each vital sign value over a period of time and a valence value of positive or negative. Trend reports may be programmed to include descriptive, trend, and clinical significance for the plurality of vital sign values. Trend reports may be programmed to communicate whether the particular vital is stable, going up, or down over a period of time. Trend reports can also include a current state of each vital sign value over a period of time and a valence value of positive or negative. For example, trend reports can include a median value of sleep duration for 2 weeks is 5 hours and 35 minutes which is 23% reduced over the last 2 weeks.

In an embodiment, the attention stratification logic 126 is programmed to have a first clinical self-reported assessments over a period of time, such as a week, after a first clinical visit of a patient. After the first week, the attention stratification logic 126 is programmed to compute trends based on the plurality of vital sign values in the first clinical self-reported assessments. As a result, the attention stratification logic 126 is programmed to determine the trend reports to monitor whether the plurality of vital sign values are stable, going up or down to determine severity associated with the plurality of vital sign values. In particular, the attention stratification logic 126 is programmed to determine and report whether a vital sign value is positive or negative for the patient. For example, trend reports shows a patient has a moderate severity of bipolar disorder as the patient has a PHQ-9 score of 11 on September 22, 2021 which is increased from a PHQ-9 score of 8 on September 1, 2021.

In an embodiment, the attention stratification logic 126 is programmed to sort patient population based on self-reports and passive sensing. In particular, the patient population can be sorted into four categories with various attention scores: 1) absence of concerning signals for an attention score of "1", 2) take note/lower risk anomalies or scores for an attention score of "2", 3) urgent/higher risk anomalies or scores for an attention score of "3", and 4) emergency/in need of attention for an attention score of "4". In an embodiment, the absence of concerning signals category is for patients who need no or minimal attention from a clinician based on a recent clinical assessments in trend reports of the patient. In particular, the recent clinical assessments can be stored as a configuration value that can be retrieved and used by the attention stratification logic 126. For example, the trend reports shows a patient has a low severity of bipolar disorder and an attenuation score of "1/4" for a PHQ-9 score of 0 in the recent clinical assessments. In an embodiment, the take note/lower risk anomalies or scores category is for patients who need low attention from the clinician based on a recent clinical assessments in trend reports of the patient. The clinician can take note of the patient for clinical assessments in the future. For example, the trend reports shows a patient has a medium severity of bipolar disorder and an attenuation score of "2/4" for a PHQ-9 score of 15 in the recent clinical assessments. In an embodiment, the urgent/higher risk anomalies or scores category is for patients who need some attention from the clinician based on a recent clinical assessments in trend reports of the patient. For example, the trend reports shows a patient has an urgent severity of bipolar disorder and an attenuation score of "3/4" for a PHQ-9 score of 25 in the recent clinical assessments. In an embodiment, the emergency/in need of attention is for patients who need great attention from the clinician by actively alerting the clinician based on a recent clinical assessments in trend reports of the patient. For example, the trend reports shows a patient has an emergent severity of bipolar disorder and an attenuation score of "4/4" for an Altman Mania Scale score of 20 in the recent clinical assessments.

In an embodiment, the attention stratification logic 126 is programmed to group patients according to anomalies or scores and/or to rank the anomalies and scores. In an embodiment, the attention stratification logic 126 is programmed to add a descriptive summary statement to highlight part of the trend reports of the patient, such as "low sleep", "continued low sleep, activity", "May not be necessary for category 1 /okay", etc. In an embodiment, the attention stratification logic 126 is programmed to determine an attention level for a patient.

In an embodiment, the attention stratification logic 126 is programmed to receive the following data items to clinicians automatically under stored program control. Data that is received from clinicians can be used to improve machine learning models of the attention stratification logic 126 because they provide feedback signals specifying what clinical action was taken based on the attention stratification of the disclosure. Although the attention stratification logic 126 does not randomly assign patients to categories concerning emergent conditions, but rather use a biased approach as represented in the heuristics discussed above, feedback signals from clinicians can serve as valuable labels to inform stratifying patients in the future.

In an embodiment, all patients stratified as Urgent and Emergent will result in an encounter between a clinician and the patient. For each encounter, the clinician may create documentation in EHR systems, including selecting from a structured data menu of encounter outcomes. In one embodiment, the attention stratification application is integrated with the EHR system. Data received from the EHR system is obtained via calls to an integration interface. In an embodiment, a clinician can select an outcome option based on a predetermined list. For example, one output option can be "patient evaluated: safe and stable" or "patient evaluated: safe but more symptomatic", etc. An example of the outcome options is shown in Table 4 below.

**Table 4. An example of the outcome options**

| Outcome # | Outcome |
|---|---|
| 1 | Rocky Mountain Crisis Line evaluated patient |
| 2 | Patient evaluated: Safe and stable. |
| 3 | Patient evaluated: Safe but more symptomatic. |
| 4 | Welfare check initiated (For emergent patients only): a) unable to contact patient, b) patient needing in-person assessment of risk, c) patient decompensating or suicidal, unable/refusing to go to emergency room, d) 911 contact date. |
| 5 | Patient referred to Emergency Room: a) patient at risk for self-harm or harming others, b)patient decompensating/gravely disabled, c) family member/friend confirmed that they would take patient (Name person and relationship to patient), d) 911 contacted for transport. |
| 6 | False alarm: a) mistake in completing the questionnaire, b) misplaced phone therefore no movement registered, c) patient lost phone, but reached via landline, d) patient with chronic thoughts of suicide but stable, e) other, with free text area. |

### 3 PROCEDURAL OVERVIEW

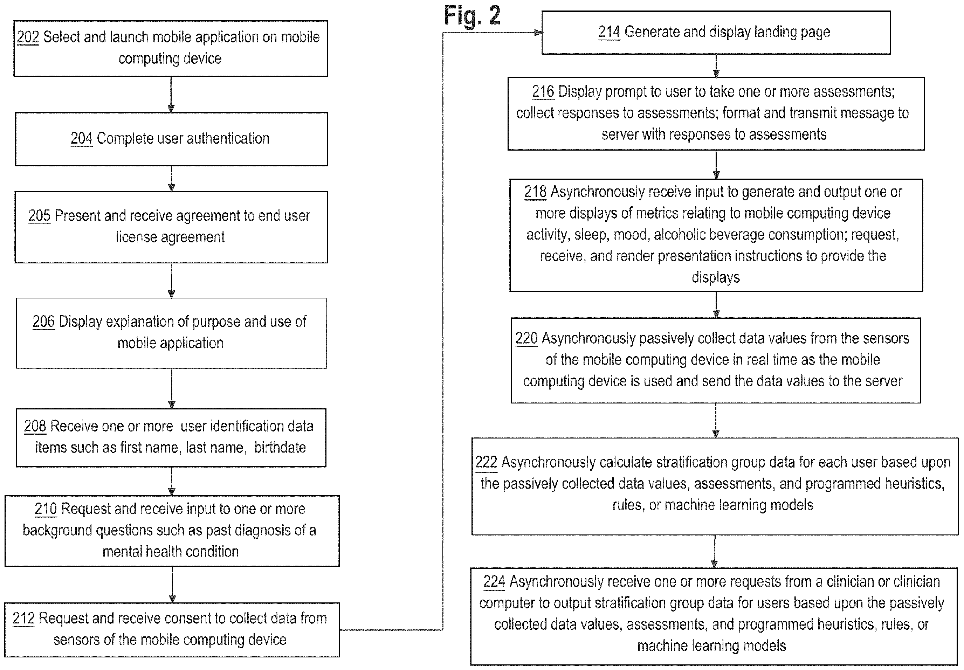

FIG. 2 illustrates a flow chart of a method of determining stratification group data for a patient in accordance with one or more embodiments. FIG. 2 can be programmed to implement a general workflow to apply one or more machine learning models to asynchronously calculate the stratification group data for the patient based on passively collected data values, assessments, and programmed heuristics, programmed rules, and model thresholds. One or more blocks in FIG. 2 may be performed by one or more components as described in FIG. 1; for example, the attention stratification logic 126 can be programmed, using one or more sequences of instructions, to execute an implementation of FIG. 2. While the various blocks in FIG. 2 are presented and described sequentially, one of ordinary skill in the art will appreciate that some or all the blocks may be executed in different orders, may be combined or omitted, and some or all the blocks may be executed in parallel. Furthermore, the blocks may be performed actively or passively.

FIG. 2 and each other flow diagram herein is intended as an illustration at the functional level at which skilled persons, in the art to which this disclosure pertains, communicate with one another to describe and implement algorithms using programming. The flow diagrams are not intended to illustrate every instruction, method object or sub-step that would be needed to program every aspect of a working program, but are provided at the same functional level of illustration that is normally used at the high level of skill in this art to communicate the basis of developing working programs.

FIG. 2 illustrates an example of user interaction with an example attention stratification application. For example, FIG. 2 can represent programmatic interactions of the mobile computing devices 102, 104 with the attention stratification logic 126 hosted at application server computer 110 (FIG. 1). In an embodiment, block 204 to block 212 inclusive can be programmed as an onboarding process for the user. In an embodiment, certain functions of block 204 to block 212 are executed once when the user executes the mobile app 103 for the first time, and in later invocations of the mobile app, executing one or more of block 204 to block 212 can be skipped.

At block 202, mobile computing device 102 selects and launches a mobile application on the mobile computing device, for example, mobile app 103. In an embodiment, the computer-implemented method can receive an MBC assessment request from a patient. In response to the MBC assessment request from the patient, the patient can use the mobile computing device 102 to select and launch a mobile application on the mobile computing device for assessing a plurality of MBC assessment data.

At block 204, the mobile app 103 executes user authentication functions, and the user of the mobile computing device 102 completes user authentication. As an example, mobile app 103 can be programmed to transmit an app protocol message to authentication logic of application server computer 110, or a third-party service, to cause transmitting an authentication code to a mobile phone number of the user, to generate and cause displaying a prompt to receive the authentication code, to validate the authentication code, and to continue execution of the mobile app. Other authentication techniques such as username and password or two-factor authentication can be used in various embodiments.

At block 205, in some embodiments, the mobile app 103 can be programmed to display, via a display device of the mobile computing device 102, an end user license agreement with prompts for the user to view and manifest acceptance of the agreement.

At block 206, in an embodiment, the mobile app 103 can be programmed to display, via a display device of the mobile computing device 102, an explanation of the purpose and use of the mobile app. Blocks 205, 206 are optional and not required in all embodiments.

At block 208, the mobile app 103 can be programmed to generate and cause displaying one or more prompts to cause the mobile computing device 102 to submit basic user identification. Example data includes first name, last name, and birthdate of the user. The basic user identification received at block 208 can facilitate retrieving related data from one or more EMR systems, if they have been made accessible, and using appropriate security and privacy protocols and measures.

At block 210, the mobile app 103 can be programmed to generate and cause displaying one or more prompts to cause the mobile computing device 102 to request and receive input to one or more background questions such as past diagnosis of a mental health condition. For example, a prompt question can be programmed as: "Have you been diagnosed with any of the following?" The available choices for data input can be bipolar disorder, anxiety, depression, obsessive compulsive disorder (OCD), postpartum depression, substance use disorder.

At block 212, the mobile app 103 can be programmed to generate and cause displaying one or more prompts to cause the mobile computing device 102 to consent to collect data from sensors of the mobile computing device. For example, block 212 can be programmed to call system services of an operating system of the mobile computing device 102 to request the user to grant permission for the mobile app 103 to access hardware elements or software services or system data of the user computer. Examples include gyroscope data, location data, location tracking services, health data, map data or route data. In an embodiment, after block 212, mobile app 103 initiates passive collection of data from sensors. In some embodiments, block 212 can be programmed in mobile app 103 to display a plurality of graphical permissions toggle switch widgets, each of which can be selected to signal that the user agrees or declines to give permission for the use of a particular kind of sensor that corresponds to each toggle switch. In an embodiment, a Continue link or the equivalent, to advance beyond block 212, is disabled until the user selects a choice for all the graphical toggle switches.

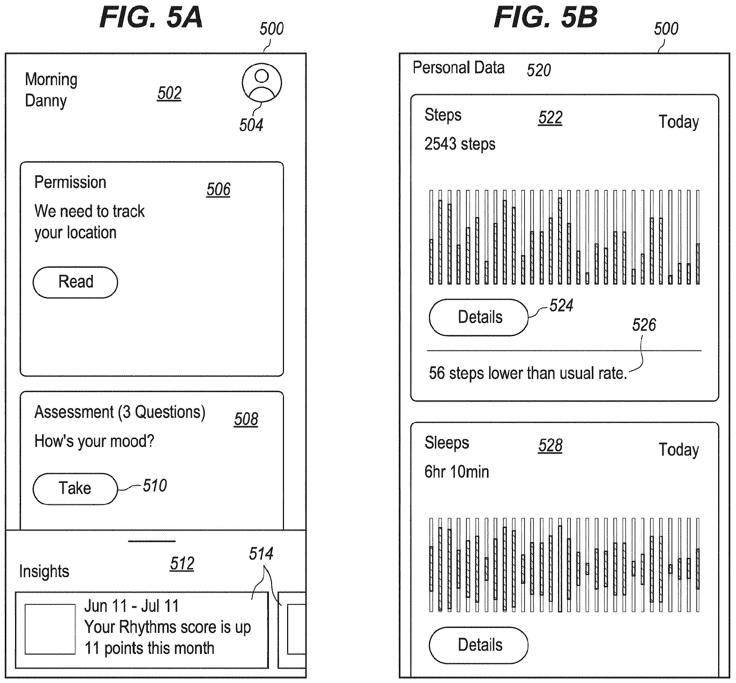

At block 214, the mobile app 103 can be programmed to generate and display a landing page via a display device of the mobile computing device 102. The form and content of the landing page can range from simple to elaborate. In an embodiment, the landing page displays basic metadata such as "Latest updates: 11:22 pm; Average mood score: 7", or similar values. For more information regarding the landing page, see FIG. 5A and the accompanying description.

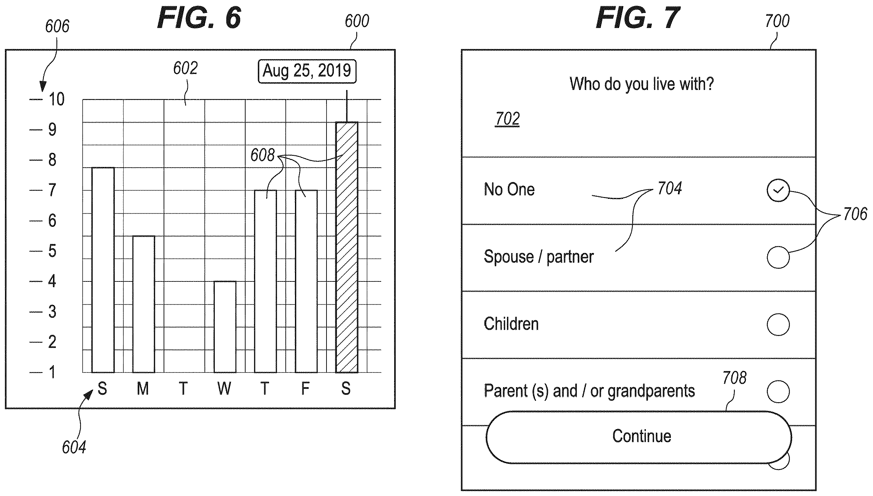

At block 216, execution of the mobile app 103 can include prompting to complete an assessment, to collect response data, and transmit the response data to the application server computer 110. For example, the application server computer 110 can be programmed to specify one or more assessments, tests, questionnaires, or other inquiries. The mobile app 103 can display an assessment interface to present one or more questions to the user, prompt for responses, and transmit the responses to the attention stratification logic 126. For more information regarding the assessment interface, see FIG. 7 and the accompanying description.

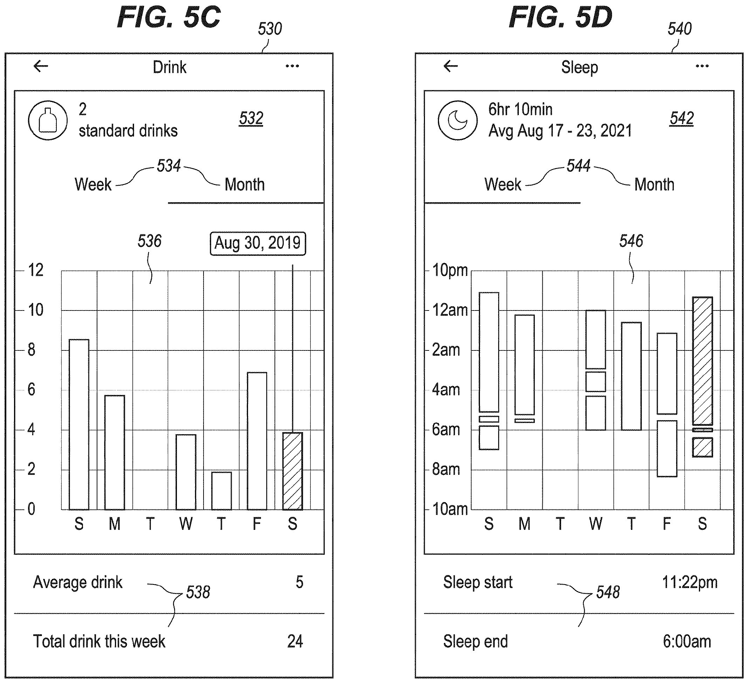

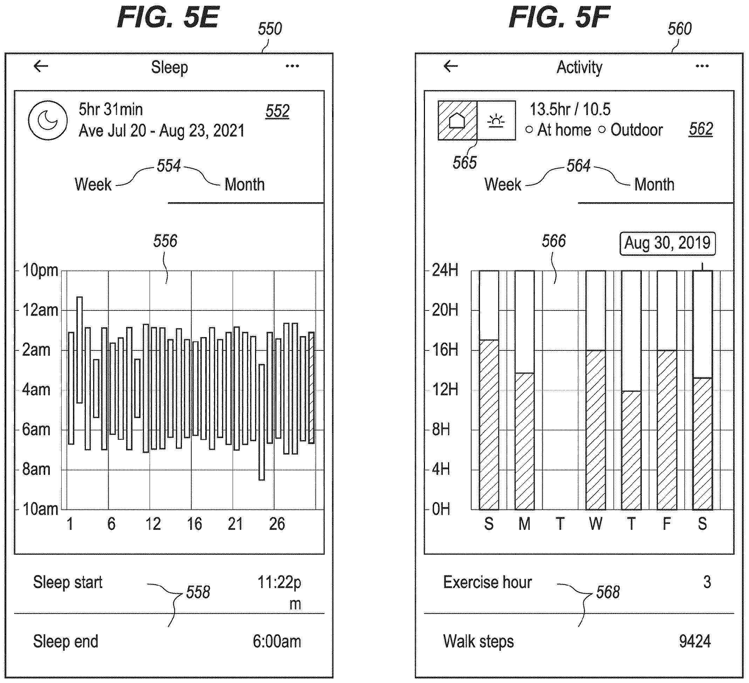

At block 218, the mobile app 103 can be programmed to asynchronously receive input to generate and output one or more displays of metrics relating to mobile computing device activity, sleep, mood, alcoholic beverage consumption; request, receive, and render presentation instructions to provide the displays. For example, the insights carousel 512 is programmed as a carousel UI widget having one or more carousel panels 514 that graphically display one of a plurality of different metrics that have been calculated for the user based upon previously passively collected data items and one or more heuristics, programmed rules, and/or machine learning models. As another example, a carousel panel 514 reports that a health score or mood score for the user has increased 11 points. Other messages for any of the other metrics of this disclosure may be presented in other carousel panels. In an embodiment, the mobile app 103 can be programmed to generate data displays relevant to mental health conditions other than those displayed here, including displays based on any of the data sensing, assessments, calculated scores, and other aspects of any other section of this disclosure. For more information regarding the graphical user interface to generate and output one or more displays of metrics, see FIG. 5B, FIG. 5C, FIG. 5D, FIG. 5E, FIG. 5F, and FIG. 6 and the accompanying descriptions.

At block 220, the mobile app 103 can be programmed to asynchronously passively collect data values from the sensors of the mobile computing device in real time as the mobile computing device is used and send the data values to the server. Examples of data that is collected are provided in other sections herein.

At block 222, in an embodiment, the attention stratification logic 126 can be programmed to asynchronously calculate stratification group data for each user based upon the passively collected data values, assessments, and programmed heuristics, rules, or machine learning models.

At block 224, the process is programmed to asynchronously receive one or more requests from a clinician or clinician computer 106 to output stratification group data for users based upon the passively collected data values, assessments, and programmed heuristics, rules, or machine learning models.

### 4. IMPLEMENTATION EXAMPLES

In an embodiment, a combination of self-reported data from patients and signals from passive sensing data from patient mobile devices are programmatically combined to generate clinical self-reported assessments to assess each patient's mental health. In an embodiment, the attention stratification logic 126 is programmed to sort patient records according to the level of attention that they need from the clinical team. In one embodiment, sorting is influenced primarily on self-reported responses to questionnaires that are electronically presented, such as the PHQ. Additionally or alternatively, one or more machine learning models can evaluate passively collected signals and output a prediction of a stratification group to which the patient record should be assigned.

In an embodiment, the attention stratification logic 126 is programmed to combine clinical heuristics, which are empirically derived where possible, anomalies and insight models to output a prediction of when to prompt self-report assessments and how to stratify a group of patients by clinical need. In this context, heuristics can mean programmed rules such as thresholds for inference values set either through empirical analysis of existing datasets or by using programmed clinical thresholds. As an example, if a patient has had median sleep of less than 3 hours over the past 2 days, a particular stratification level could be indicated. Furthermore, the attention stratification logic 126 can be programmed to identify anomalies as a change in time series data that is statistically different to what it should be, based on a past period or baseline.

In an embodiment, the attention stratification logic 126 is programmed to use Insight Scores in the form of models that provide an estimate of a patient's mental health status. A machine learning model that has been trained using a training dataset consisting of responses of others to the PHQ questionnaire can be used. Insight Scores can be associated with any specified clinical condition, such as depression. In an embodiment, the attention stratification logic 126 is programmed to generate Insight Scores for sleep, social, routine, physical activity and overall condition. Models to assess sleep, social, routine, and physical activity receive, as input, inferences that are deemed to be a measure of the overarching category. For example, a social insight model can be programmed to receive all location inferences as inputs and can be trained on an individual element of the PHQ.

### 4.1 COLLECTING DATA FROM PATIENTS

In an embodiment, the attention stratification logic 126 is programmed to collect various data items from patients. For example, the attention stratification logic 126 is programmed to continuously collect passive sensing data. As another example, the attention stratification logic 126 is programmed to collect onboarding data for basic information and self-assessments, such as PHQ-8, GAD-7, Altman Mania Scale, Sleep questions (for the first 2 weeks), of a patient. There are many types of independent triggers and a trigger orchestrator. In an embodiment, the attention stratification logic 126is programmed to execute a trigger orchestrator that can iterate on versions of each independent trigger and add or remove triggers as appropriate. The trigger orchestrator can be programmed to detect and receive the results of all the independent triggers and decide what to push to each member. The trigger orchestrator can be programmed to enforce constraints such as minimum and maximum frequency per SR. In an embodiment, the attention stratification logic 126 is programmed to request patients to respond to an assessment on a corresponding schedule, such as PHQ-8: no closer than 7 days but at least every 14 days, GAD-7: no closer than 7 days but at least every 14 days, Altman Mania Scale: no closer than 7 days but at least every 14 days, sleep question: to be asked a few times soon after onboarding. In an embodiment, the attention stratification logic 126 is programmed to request patients to respond to an assessment without subject frequency constraints.

In an embodiment, the attention stratification logic 126 is programmed to collect a plurality of patient data items from a patient in a three-step procedure automatically under stored program control. In particular, the three-step procedure include 1) an initial clinical assessment during the first week based on clinical need and patient population, 2) random clinical assessments in weeks 2-6, and 3) random clinical assessments in weeks 6 and over. For example, when a patient schedule a first visit to a clinic, a clinician can use the clinician computer 106 to setup the three-step procedure for the patient. In particular, the clinician can have the first assessment for the patient during the first week when the patient visit the clinic based on the schedule. The day of delivery of the assessments is configurable, to spread them out as desired on different days. The clinician can provide one or more clinical assessments, such as PHQ-9 for depression and bipolar disorder, GAD-7 for anxiety, depression, and bipolar disorder, and Altman Mania Scale for bipolar disorder. The clinician can also ask the patient a plurality of questions, such as sleep tuning questions, associated with the patient's health status and bio information during the first two weeks. Likewise, the clinician can collect basic information of the patient. An example of three-step procedure to collect patient information is shown in Table 5 below. An example of questions to collect patient bio information is shown in Table 6 below.

**Table 5. An example of three-step procedure to collect patient information.**

| Week# | Step | Example clinical assessments |
|---|---|---|
| 1 | Initial clinical assessment | PHQ-9 (for depression and bipolar disorder); GAD-7 (for anxiety, depression and bipolar disorder); Altman Mania Scale (for bipolar disorder) |
| 2-6 | Random clinical assessments | PHQ-9: every 14 days |
| | | GAD-7: every 14 days |
| | | Altman Mania Scale: every 14 days |
| 6+ | Random clinical assessments | PHQ-9: every 14 days |
| | | GAD-7: every 14 days |
| | | Altman Mania Scale: every 14 days |

**Table 6. An example of questions to collect patient bio information.**

| Question # | Question for the patient | Example clinical assessments |
|---|---|---|
| 1 | Patient's age and sex | values sometimes can be determined from other systems that were involved in enrolling the patient in a larger system in which the attention stratification application forms a part |
| 2 | Who lives with the patient? | Children/Spouse |
| 3 | Patient's work status | None/Part-time/Full-time |
| 4 | Patient's school status | None/High School/College |
| 5 | Phone carrying consistency | Sometimes/Frequently/Always |
| 6 | Does the patient ever work night shifts? | Never/Sometimes/Always |
| 7 | Is the patient frequently driving or flying while working, excluding commuting? | Rarely/Sometimes/Usually |
| 8 | Does the patient spend more than one month per year at a residence other than your home? | No/Yes. If yes, how much of the year |

In an embodiment, the attention stratification logic 126 is programmed to carry out random clinical assessments during weeks 2-6. The random clinical assessments during weeks 2-6 can be triggered using clinical heuristics, such as a bipolar patient who is suddenly not sleeping. For example, a clinical organization can configure which assessments are pushed based on the patient's diagnosis. As another example, a clinician can ask the patient a check-in question, such as "how are you doing?". In order to mitigate bias in self-reporting prompting, the attention stratification logic 126 is programmed to randomly prompt the patient to complete the same self-reports every a predetermined period of time, such as a week or two weeks. To avoid oversaturation of assessment delivery, the attention stratification logic 126 is programmed to store a configurable minimum number of days between delivery of a given assessment. In an embodiment, the attention stratification logic 126 is programmed to suppress delivery of an otherwise configured assessment if the minimum number of days has not elapsed. If the patient has not received a clinical assessment, the attention stratification logic 126 is programmed to implement a configurable minimum frequency at which a clinical assessment should be delivered. For example, the attention stratification logic 126 is programmed to implement a clinical assessment for PHQ-9, GAD-7, or Altman Mania Scale every 14 days. In an embodiment, the attention stratification logic 126 is programmed to suppress certain assessments so that, with the exception of assessments that are triggered from clinical heuristics, the patient does not receive more than one assessment on a given day. In an embodiment, the attention stratification logic 126 is programmed to spot-check inference accuracy such as the accuracy of sleep start time and end time. In an embodiment, the attention stratification logic 126 is programmed to initiate such checks randomly and based on clinical heuristics and anomalies. In an embodiment, the attention stratification logic 126 is programmed to initiate such checks using the same default assessment period for the related clinical assessment, for example, PHQ every 14 days.

In an embodiment, the attention stratification logic 126 is programmed to carry out random clinical assessments after week 6. In particular, the random clinical assessments after week 6 can be triggered by three factors: 1) passive data anomaly detection, 2) clinical heuristics, and 3) clinician. For example, when the random clinical assessments after week 6 are triggered by passive data anomaly detection, the attention stratification logic 126 is programmed to deliver a set of assessments corresponding to diagnosis from passive data anomaly detection. As another example, when the random clinical assessments after week 6 are triggered by clinical heuristics, such as a bipolar patient who is suddenly not sleeping, the attention stratification logic 126 is programmed to carry out assessments based on clinical population. As another example, when the random clinical assessments after week 6 are triggered by the clinician, the attention stratification logic 126 is programmed to carry out assessments based on clinician's diagnosis. In an embodiment, the attention stratification logic 126 is programmed to include a "how are you doing" check-in question at the time that this assessment is transmitted. In an embodiment, the attention stratification logic 126 is programmed to carry out assessments with a frequency which is not more than once per 7 days. Likewise, the patient should not receive more than one assessment on a given day. To avoid oversaturation of assessment delivery, the attention stratification logic 126 is programmed to use a configurable minimum number of days between delivery of a given assessment. If patient has not received a clinical assessment, the attention stratification logic 126 is programmed to use a configurable minimum frequency at which a given assessment should be delivered. For example, the attention stratification logic 126 is programmed to implement a clinical assessment for PHQ-9, GAD-7, or Altman Mania Scale every 14 days. With exception of triggered from passive data anomaly and clinical heuristics, the attention stratification logic 126 is programmed to cause the patient not to receive more than one assessment on a given day.

### 4.2 DELIVERING DATA TO CLINICIANS

In an embodiment, the attention stratification logic 126 is programmed to output vitals and assessments reporting to clinicians. n an embodiment, the attention stratification logic 126 is programmed to output attention Sorting based on self-reports and passive sensing to clinicians. In particular, the patient population can be sorted into four different categories: 1) absence of concerning signals, 2) take note / anomalies or scores, 3) urgent/higher risk anomalies or scores in need of attention, and 4) emergency/in need of attention for actively alerting clinicians.

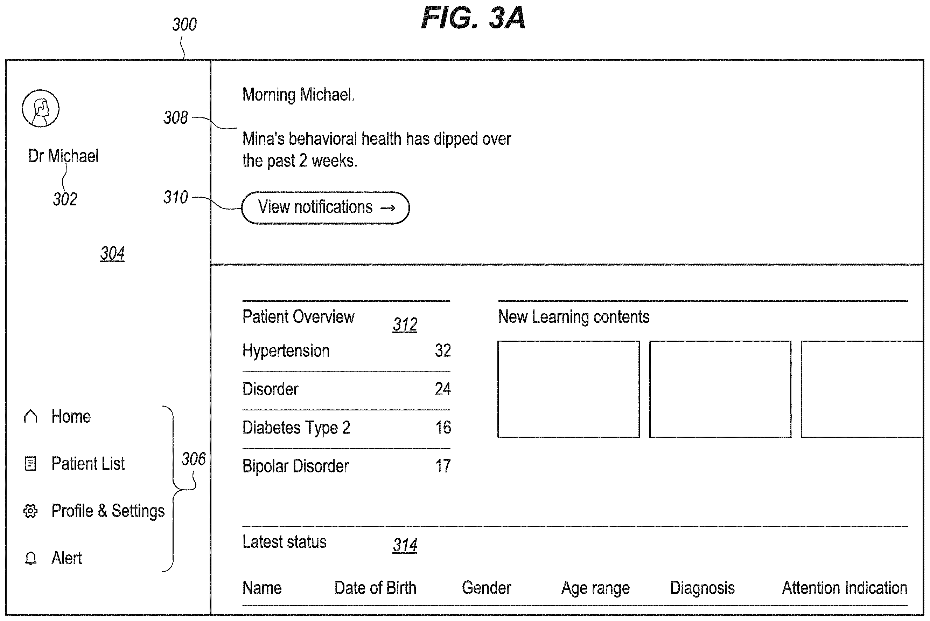

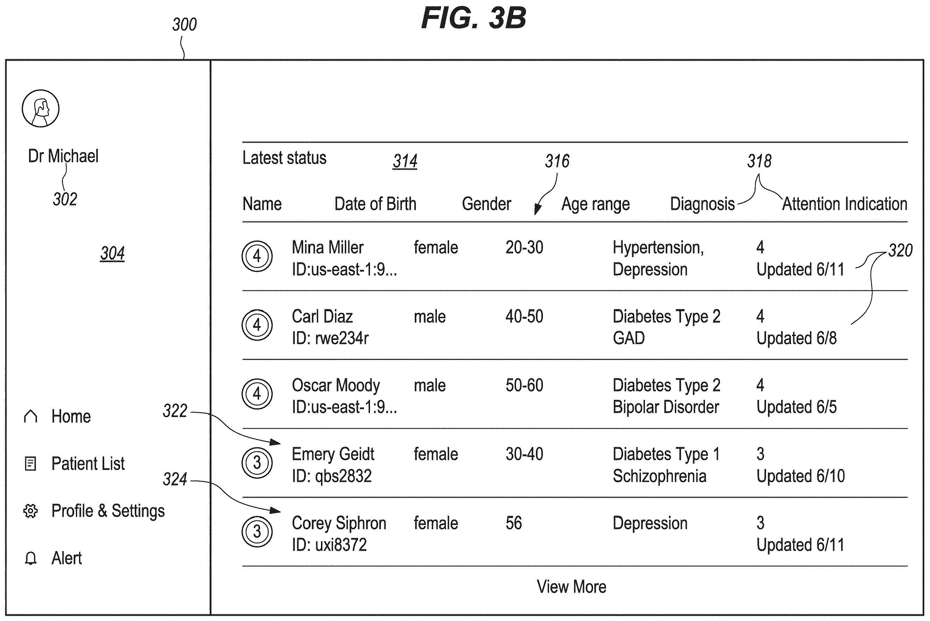

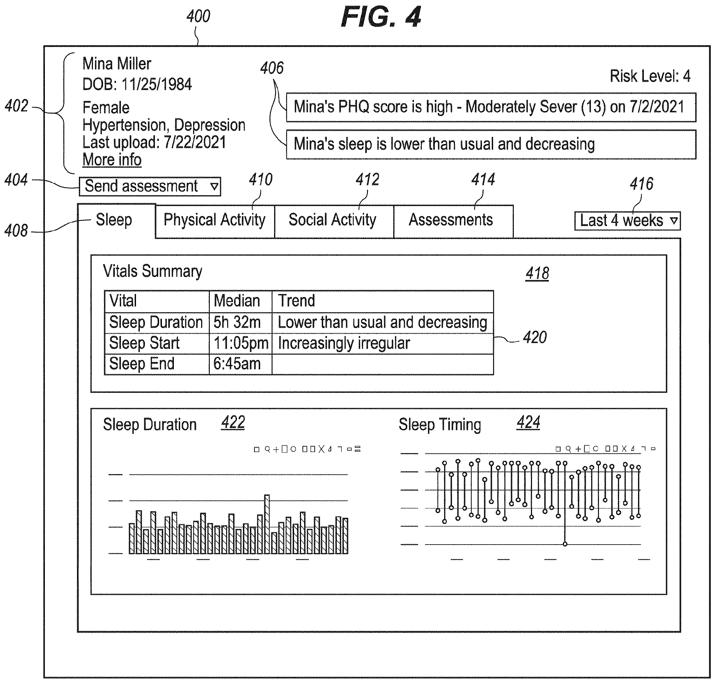

In an embodiment, the attention stratification logic 126 is programmed to generate presentation instructions 114 which when rendered via a user computer can cause displaying data supplied to clinicians in the form of the graphical user interfaces of FIG. 3A, FIG. 3B, and FIG. 4. FIG. 3A and FIG. 3B illustrate examples of user interface of data for a clinician in accordance with one or more embodiments. FIG. 3A illustrates an example user interface of data for a clinician. In an embodiment, a computer display device 300 of the clinician computer 106 can render and display a graphical user interface based on presentation instructions 114 and having a notification panel 308, user identification panel 304, function links 306, overview panel 312, and status panel 314. The GUI of FIG. 3A is personalized for a particular clinician, by using security controls embedded in queries to retrieve and display data only for records of patients that are associated, in the database 112, with a particular clinician.

In an embodiment, the notification panel 308 shows data based on queries to database 112 to retrieve one or more records of users of mobile computing devices 102, 104 that are grouped in higher strata, groups, or categories. For example, the presentation instructions 114 can be programmed with an embedded SELECT query directed to database 112 to retrieve records of patients with the top *N* scores for behavioral health or other metrics and to select and display a notification in the panel for one or more patients. In an embodiment, notification panel 308 comprises a view link 310 that is programmed, when selected, to cause rendering a further page of notifications for other patients.

In an embodiment, the user identification panel 304 comprises a clinician identifier 302 specifying a name or display name of a clinician who is then currently using the clinician computer 106 and associated with patient records for which data is displayed in other parts of the GUI. In an embodiment, the function links 306 are programmed as active links which, when selected, transmit URLs or other requests to the attention stratification logic 126 and cause the presentation instructions 114 to generate and return display instructions for other pages associated with other functions. Examples include generating and displaying a list of patients of the clinician identified by clinician identifier 302, accessing a profile of the clinician and settings such as name, image, password or other account details, and viewing a list of alerts or notifications.

In an embodiment, the overview panel 312 comprises a list of patient diagnoses and counts of numbers of patients that are associated with those diagnoses. The overview panel 312 can be generated by a SELECT query to the database 112 that is formatted to retrieve records of all patients that are associated with the clinician, sort by the values in a diagnosis column of each record and calculate counts of the values for each diagnosis.

In an embodiment, FIG. 3B represents the same GUI as FIG. 3A in which the display has been scrolled to show all of the status panel. Referring now to FIG. 3B, in an embodiment, the status panel 314 comprises a table 316 in which rows 320 represent individual patients of the clinician shown in clinician identifier 302, and columns 318 specify patient attributes and an attention indication. Rows 322, 324 show example values for two different patients. In one embodiment, columns 318 can specify patient name, gender, age or age range, diagnosis, and attention indication. In other embodiments, other patient attributes can be shown with the attention indication; while the patient attributes can be retrieved from column values of patient records in database 112 and are relatively static, the attention indication is dynamically calculated periodically and stored in a column value of the database record periodically. Calculations of attention indications can occur hourly, weekly, nightly, weekly, or according to other programmed schedules, or in response to the attention stratification logic 126 detecting an update to a data value that is obtained from the mobile computing devices 102, 104. In an embodiment, selecting a patient row in FIG. 3B signals the attention stratification logic 126 to generate and format presentation instructions 114 to enable rendering and displaying detailed analytical data for an individual patient in the display device 300 of the clinician computer 106.

FIG. 4 illustrates an example graphical user interface in the display device for an individual patient in accordance with one or more embodiments. In the example of FIG. 4, a GUI 400 comprises patient identifying information 402, which can be rendered after queries to database 112 to retrieve column values for name, date of birth, gender, diagnoses, and last date of a data upload. The GUI 400 can comprise a function widget 404 that is programmed as a pull-down menu to access data management or transmission functions, such as sending an assessment of the patient to another account or computer. The GUI 400 can comprise one or more notification messages in notification boxes 406 that specify one or more conditions of greatest concern that have been calculated for the specified patient.

In an embodiment, presentation instructions 114 can be programmed to render the GUI 400 with graphical tabs such as a sleep tab 408, physical activity tab 410, social activity tab 412, and assessments tab 414. Each of the sleep tab 408, physical activity tab 410, and social activity tab 412 can be programmed to render one or more charts or graphs that show data that has been collected from one of the mobile computing devices 102, 104 of the patient and stored in database 112. For example, sleep tab 408 can comprise a vitals summary panel 418 having a table 420 having rows for a plurality of different vital attributes. Columns of table 420 can be programmed to show attributes of each of the vital values, the median magnitude of the data of the patient for a corresponding vital, and a trend indicator for the same corresponding vital. In an embodiment, sleep tab 408 can comprise a sleep duration bar chart 422 comprising graphical bars that represent values of the magnitude of sleep time for the patient for different periods, such as days. In an embodiment, sleep tab 408 can comprise a sleep timing plot 424 that can show the hours, within a given day or night, during which the patient was asleep.

In some embodiments, the GUI 400 may comprise a scope widget 416 that is programmed as a pull-down menu having values to specify different ranges of data for the patient; the scope widget can be programmed, in response to input from the clinician computer 106 to change the pull-down menu to a different range value, to transmit a query based on the range value to database 112 to retrieve data for the patient corresponding to the range value and re-render the GUI 400 using data values within the changed range. In this manner, the GUI 400 can be dynamically updated to show data within different periods of time or ranges.

Turning to FIG. 5A, FIG. 5A illustrates an example graphical user interface which includes a landing page in accordance with one or more embodiments. In an embodiment, a display device 500 of a mobile computing device 102 comprises a landing page 502 having a greeting or notification, user profile icon 504, permission panel 506, assessment panel 508, and insights carousel 512. The greeting or notification is optional. The user profile icon 504 can comprise a generic representation of a user or can include a thumbnail graphical image that the user has uploaded or that the system has otherwise acquired. The permission panel 506 is optional and can be programmed to be presented only if the user has not previously granted location tracking permission. When present, in an embodiment, the permission panel 506 is programmed with an active link which, when selected, causes displaying information about location tracking and a prompt for the user to signal approval for the mobile computing device 102 to report location data to the application server computer 110. In response to the signal, the mobile app 103 can be programmed to call system services of an operating system of the mobile computing device 102 to authorize transmission of location data.

In an embodiment, the assessment panel 508 is programmed to specify one or more assessments, tests, questionnaires, or other inquiries. The assessment panel 508 can be programmed to display an active link 510 which, when selected, signals the mobile app 103 to present one or more questions to the user, prompt for responses, and transmit the responses to the attention stratification logic 126. For example, in FIG. 5A, in an embodiment, the insights carousel 512 is programmed as a carousel UI widget having one or more carousel panels 514 that graphically display one of a plurality of different metrics that have been calculated for the user based upon previously passively collected data items and one or more heuristics, programmed rules, and/or machine learning models. In the example of FIG. 5A, a carousel panel 514 reports that a health score or mood score for the user has increased 11 points. Other messages for any of the other metrics of this disclosure may be presented in other carousel panels.

Turning to FIG. 5B, FIG. 5B illustrates an example graphical user interface for display of personal data via the mobile app in accordance with one or more embodiments. In the example of FIG. 5B, display device 500 has rendered a personal data panel 520 that comprises a step count sub panel 522 and a sleep data sub panel 528. The step count sub panel 522 can be programmed to display a bar graph showing a number of steps through which the mobile computing device 102 traversed over a set of days, weeks, months, or other periods. The step count sub panel 522 can be programmed to display an active link 524 titled Details which, when selected, causes the mobile app 103 to update the personal data panel 520 to show detailed data relating to step counts such as number of steps for a particular day and times in which steps were recorded.

Similarly, sleep data sub panel 528 can be programmed to display a bar graph showing periods during which the mobile computing device 102 has been stationery and had no user interaction, and therefore was associated with sleep, over a set of days, weeks, months, or other periods. The sleep data sub panel 528 can be programmed to display an active link titled Details which, when selected, causes the mobile app 103 to update the personal data panel 520 to show detailed data relating to sleep events such as the hours of sleep for a particular day and times in which sleep was recorded.

FIG. 5C illustrates an example graphical user interface which shows consumption of alcoholic beverages by a user of a mobile computing device in accordance with one or more embodiments. In an embodiment, display device 530 has received and rendered presentation instructions to show a user interface 532 comprising a bar chart 536 illustrating numbers of drinks that the user of the mobile computing device 102 has reported to the mobile app 103. The vertical axis can indicate number of standard drinks and the horizontal axis can illustrate days within a week. In an embodiment, user interface 532 comprises two or more selectable tabs 534 which, when selected, can cause the mobile app 103 to update the UI to show different bar charts for different periods. The user interface 532 can be programmed to display an average number of drinks 5 for the same period as shown in bar chart 536 and a total number of drinks for that period.

FIG. 5D illustrates an example graphical user interface which shows detailed data for weekly sleep periods in accordance with one or more embodiments. In the example of FIG. 5D, a display device 540 has received and rendered presentation instructions to show a user interface 542 comprising a bar plot 546 comprising graphical bars that represent periods of sleep. Bars can have gaps or breaks to represent periods of activity within larger periods of sleep. The vertical axis can indicate time of day and the horizontal axis can illustrate days within a week. In an embodiment, user interface 542 comprises two or more selectable tabs 544 which, when selected, can cause the mobile app 103 to update the UI to show different plots for different periods. Bars in the bar plot can be programmed as active links which, when selected, cause the mobile app to display specific sleep metrics 548 that correspond to the selected bar, such as time of start and time of end.

FIG. 5E illustrates an example graphical user interface which shows detailed data for monthly sleep periods in accordance with one or more embodiments. In particular, FIG. 5E illustrates the display of FIG. 5D in response to a selection of a selectable tab for data for a month. In response to a selection of a tab 544 denoted Month or the equivalent, the mobile app 103 can be programmed to update the interface 542 (FIG. 5D) in the form of user interface 552 and to display a different bar plot 556 with data for a month. Bars in the bar plot can be programmed as active links which, when selected, cause the mobile app to display specific sleep metrics 558 that correspond to the selected bar.

FIG. 5F illustrates an example graphical user interface which shows detailed data for monthly activity in accordance with one or more embodiments. In particular, FIG. 5F illustrates an example graphical user interface that can be generated and displayed in an embodiment to show periods of exercise, walk, or other activity through which the mobile computing device traversed. In an embodiment, a display device 560 of a mobile computing device 102 has received and rendered presentation instructions to show a user interface 562 comprising a bar chart 566 illustrating periods of activity during daytime or nighttime. The vertical axis can indicate time of day, and the horizontal axis can illustrate days within a week. Bars in the bar plot can be programmed as active links which, when selected, cause the mobile app to display specific activity metrics 568 that correspond to the selected bar, such as total number of hours of exercise, steps, or other activity metrics. In an embodiment, user interface 562 is programmed to display selectable icons 565 representing day or night, each of which is an active link which, when selected, signals the mobile app 103 to update the user interface 562 to show data corresponding to the selection.

Turning to FIG. 6, FIG. 6 illustrates an example graphical user interface which displays health scores of a user of a mobile computing device in accordance with one or more embodiments. In an embodiment, a display device 600 of a mobile computing device 102 has received and rendered presentation instructions to show a user interface comprising a bar graph 602 of health score values of the user. A vertical axis 606 can represent magnitude of score values, and a horizontal axis 604 can represent days of the week. Graphical bars 608 can specify score values for each period of the horizontal axis 604. In some embodiments, each of the graphical bars 608 can be rendered as an active link which, when selected, causes updating the user interface to specify a day of the week that the bar represents. Score values in bar graph 602 can be PHQ-9 scores, GAD-7 scores, mood scores, or other health values that are calculated as described in other sections herein.

Turning to FIG. 7, FIG. 7 illustrates an example assessment interface in accordance with one or more embodiments. In an embodiment, mobile app 103 is programmed to generate and display an assessment interface 702 in a display device 700 of the mobile computing device 102. The assessment interface 702 is programmed to display a question, a plurality of possible answers 704, and a corresponding set of radio buttons 706 that can be selected to signal a response. In an embodiment, assessment interface 702 comprises an active link 708 titled Continue, Submit, or similar, which when selected, causes the mobile app 103 to form an app protocol message, POST request or other message transmission from the mobile app to the application server computer 110 to transfer response data from radio buttons 706 to the application server computer for recording in database 112 and use in analytics.

### 5. HARDWARE OVERVIEW

According to one embodiment, the techniques described herein are implemented by at least one computing device. The techniques may be implemented in whole or in part using a combination of at least one server computer and/or other computing devices that are coupled using a network, such as a packet data network. The computing devices may be hard-wired to perform the techniques, or may include digital electronic devices such as at least one application-specific integrated circuit (ASIC) or field programmable gate array (FPGA) that is persistently programmed to perform the techniques, or may include at least one general purpose hardware processor programmed to perform the techniques pursuant to program instructions in firmware, memory, other storage, or a combination. Such computing devices may also combine custom hard-wired logic, ASICs, or FPGAs with custom programming to accomplish the described techniques. The computing devices may be server computers, workstations, personal computers, portable computer systems, handheld devices, mobile computing devices, wearable devices, body mounted or implantable devices, smartphones, smart appliances, internetworking devices, autonomous or semi-autonomous devices such as robots or unmanned ground or aerial vehicles, any other electronic device that incorporates hard-wired and/or program logic to implement the described techniques, one or more virtual computing machines or instances in a data center, and/or a network of server computers and/or personal computers.

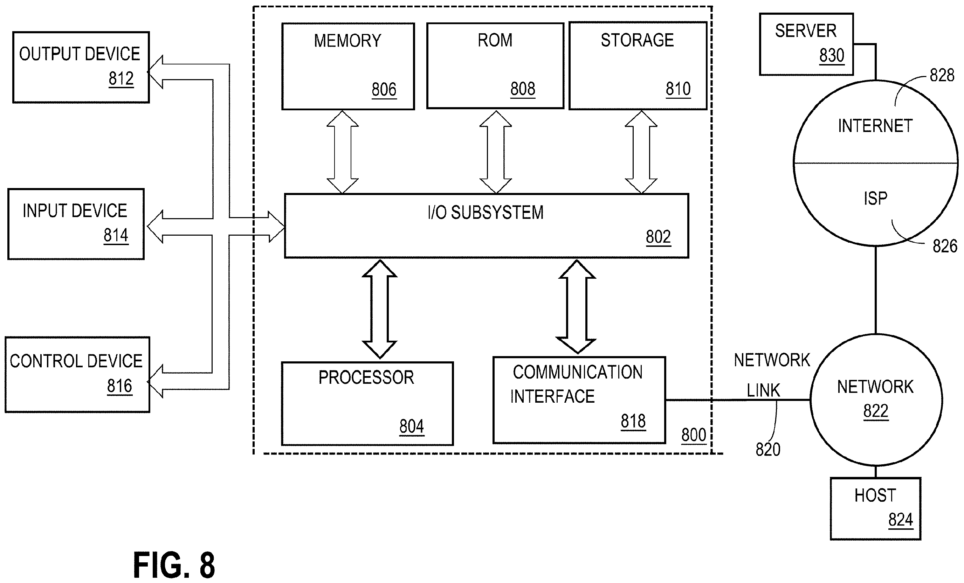

FIG. 8 illustrates a computer system in accordance with one or more embodiments. In the example of FIG. 8, a computer system 800 and instructions for implementing the disclosed technologies in hardware, software, or a combination of hardware and software, are represented schematically, for example as boxes and circles, at the same level of detail that is commonly used by persons of ordinary skill in the art to which this disclosure pertains for communicating about computer architecture and computer systems implementations.

Computer system 800 includes an input/output (I/O) subsystem 802 which may include a bus and/or other communication mechanism(s) for communicating information and/or instructions between the components of the computer system 800 over electronic signal paths. The I/O subsystem 802 may include an I/O controller, a memory controller and at least one I/O port. The electronic signal paths are represented schematically in the drawings, for example as lines, unidirectional arrows, or bidirectional arrows.

At least one hardware processor 804 is coupled to I/O subsystem 802 for processing information and instructions. Hardware processor 804 may include, for example, a general-purpose microprocessor or microcontroller and/or a special-purpose microprocessor such as an embedded system or a graphics processing unit (GPU) or a digital signal processor or ARM processor. Processor 804 may comprise an integrated arithmetic logic unit (ALU) or may be coupled to a separate ALU.

Computer system 800 includes one or more units of memory 806, such as a main memory, which is coupled to I/O subsystem 802 for electronically digitally storing data and instructions to be executed by processor 804. Memory 806 may include volatile memory such as various forms of random-access memory (RAM) or other dynamic storage device. Memory 806 also may be used for storing temporary variables or other intermediate information during execution of instructions to be executed by processor 804. Such instructions, when stored in non-transitory computer-readable storage media accessible to processor 804, can render computer system 800 into a special-purpose machine that is customized to perform the operations specified in the instructions.

Computer system 800 further includes non-volatile memory such as read only memory (ROM) 808 or other static storage device coupled to I/O subsystem 802 for storing information and instructions for processor 804. The ROM 808 may include various forms of programmable ROM (PROM) such as erasable PROM (EPROM) or electrically erasable PROM (EEPROM). A unit of persistent storage 810 may include various forms of non-volatile RAM (NVRAM), such as FLASH memory, or solid-state storage, magnetic disk or optical disk such as CD-ROM or DVD-ROM and may be coupled to I/O subsystem 802 for storing information and instructions. Storage 810 is an example of a non-transitory computer-readable medium that may be used to store instructions and data which when executed by the processor 804 cause performing computer-implemented methods to execute the techniques herein.

The instructions in memory 806, ROM 808 or storage 810 may comprise one or more sets of instructions that are organized as modules, methods, objects, functions, routines, or calls. The instructions may be organized as one or more computer programs, operating system services, or application programs including mobile apps. The instructions may comprise an operating system and/or system software; one or more libraries to support multimedia, programming or other functions; data protocol instructions or stacks to implement TCP/IP, HTTP or other communication protocols; file format processing instructions to parse or render files coded using HTML, XML, JPEG, MPEG or PNG; user interface instructions to render or interpret commands for a graphical user interface (GUI), command-line interface or text user interface; application software such as an office suite, internet access applications, design and manufacturing applications, graphics applications, audio applications, software engineering applications, educational applications, games or miscellaneous applications. The instructions may implement a web server, web application server or web client. The instructions may be organized as a presentation layer, application layer and data storage layer such as a relational database system using structured query language (SQL) or no SQL, an object store, a graph database, a flat file system or other data storage.

Computer system 800 may be coupled via I/O subsystem 802 to at least one output device 812. In one embodiment, output device 812 is a digital computer display. Examples of a display that may be used in various embodiments include a touch screen display or a light-emitting diode (LED) display or a liquid crystal display (LCD) or an e-paper display. Computer system 800 may include other type(s) of output devices 812, alternatively or in addition to a display device. Examples of other output devices 812 include printers, ticket printers, plotters, projectors, sound cards or video cards, speakers, buzzers or piezoelectric devices or other audible devices, lamps or LED or LCD indicators, haptic devices, actuators or servos.

At least one input device 814 is coupled to I/O subsystem 802 for communicating signals, data, command selections or gestures to processor 804. Examples of input devices 814 include touch screens, microphones, still and video digital cameras, alphanumeric and other keys, keypads, keyboards, graphics tablets, image scanners, joysticks, clocks, switches, buttons, dials, slides, and/or various types of sensors such as force sensors, motion sensors, heat sensors, accelerometers, gyroscopes, and inertial measurement unit (IMU) sensors and/or various types of transceivers such as wireless, such as cellular or Wi-Fi, radio frequency (RF) or infrared (IR) transceivers and Global Positioning System (GPS) transceivers.

Another type of input device is a control device 816, which may perform cursor control or other automated control functions such as navigation in a graphical interface on a display screen, alternatively or in addition to input functions. Control device 816 may be a touchpad, a mouse, a trackball, or cursor direction keys for communicating direction information and command selections to processor 804 and for controlling cursor movement on display 812. The input device may have at least two degrees of freedom in two axes, a first axis (e.g., x) and a second axis (e.g., y), that allows the device to specify positions in a plane. Another type of input device is a wired, wireless, or optical control device such as a joystick, wand, console, steering wheel, pedal, gearshift mechanism or other type of control device. An input device 814 may include a combination of multiple different input devices, such as a video camera and a depth sensor.

In another embodiment, computer system 800 may comprise an internet of things (IoT) device in which one or more of the output device 812, input device 814, and control device 816 are omitted. Or, in such an embodiment, the input device 814 may comprise one or more cameras, motion detectors, thermometers, microphones, seismic detectors, other sensors or detectors, measurement devices or encoders and the output device 812 may comprise a special-purpose display such as a single-line LED or LCD display, one or more indicators, a display panel, a meter, a valve, a solenoid, an actuator or a servo.

When computer system 800 is a mobile computing device, input device 814 may comprise a global positioning system (GPS) receiver coupled to a GPS module that is capable of triangulating to a plurality of GPS satellites, determining and generating geo-location or position data such as latitude-longitude values for a geophysical location of the computer system 800. Output device 812 may include hardware, software, firmware and interfaces for generating position reporting packets, notifications, pulse or heartbeat signals, or other recurring data transmissions that specify a position of the computer system 800, alone or in combination with other application-specific data, directed toward host 824 or server 830.

Computer system 800 may implement the techniques described herein using customized hard-wired logic, at least one ASIC or FPGA, firmware and/or program instructions or logic which when loaded and used or executed in combination with the computer system causes or programs the computer system to operate as a special-purpose machine. According to one embodiment, the techniques herein are performed by computer system 800 in response to processor 804 executing at least one sequence of at least one instruction contained in main memory 806. Such instructions may be read into main memory 806 from another storage medium, such as storage 810. Execution of the sequences of instructions contained in main memory 806 causes processor 804 to perform the process steps described herein. In alternative embodiments, hard-wired circuitry may be used in place of or in combination with software instructions.

The term "storage media" as used herein refers to any non-transitory media that store data and/or instructions that cause a machine to operation in a specific fashion. Such storage media may comprise non-volatile media and/or volatile media. Non-volatile media includes, for example, optical or magnetic disks, such as storage 810. Volatile media includes dynamic memory, such as memory 806. Common forms of storage media include, for example, a hard disk, solid state drive, flash drive, magnetic data storage medium, any optical or physical data storage medium, memory chip, or the like.

Storage media is distinct from but may be used in conjunction with transmission media. Transmission media participates in transferring information between storage media. For example, transmission media includes coaxial cables, copper wire and fiber optics, including the wires that comprise a bus of I/O subsystem 802. Transmission media can also take the form of acoustic or light waves, such as those generated during radio-wave and infra-red data communications.

Various forms of media may be involved in carrying at least one sequence of at least one instruction to processor 804 for execution. For example, the instructions may initially be carried on a magnetic disk or solid-state drive of a remote computer. The remote computer can load the instructions into its dynamic memory and send the instructions over a communication link such as a fiber optic or coaxial cable or telephone line using a modem. A modem or router local to computer system 800 can receive the data on the communication link and convert the data to a format that can be read by computer system 800. For instance, a receiver such as a radio frequency antenna or an infrared detector can receive the data carried in a wireless or optical signal and appropriate circuitry can provide the data to I/O subsystem 802 such as place the data on a bus. I/O subsystem 802 carries the data to memory 806, from which processor 804 retrieves and executes the instructions. The instructions received by memory 806 may optionally be stored on storage 810 either before or after execution by processor 804.

Computer system 800 also includes a communication interface 818 coupled to bus 802. Communication interface 818 provides a two-way data communication coupling to network link(s) 820 that are directly or indirectly connected to at least one communication networks, such as a network 822 or a public or private cloud on the Internet. For example, communication interface 818 may be an Ethernet networking interface, integrated-services digital network (ISDN) card, cable modem, satellite modem, or a modem to provide a data communication connection to a corresponding type of communications line, for example an Ethernet cable or a metal cable of any kind or a fiber-optic line or a telephone line. Network 822 broadly represents a local area network (LAN), wide-area network (WAN), campus network, internetwork or any combination thereof. Communication interface 818 may comprise a LAN card to provide a data communication connection to a compatible LAN, or a cellular radiotelephone interface that is wired to send or receive cellular data according to cellular radiotelephone wireless networking standards, or a satellite radio interface that is wired to send or receive digital data according to satellite wireless networking standards. In any such implementation, communication interface 818 sends and receives electrical, electromagnetic or optical signals over signal paths that carry digital data streams representing various types of information.

Network link 820 typically provides electrical, electromagnetic, or optical data communication directly or through at least one network to other data devices, using, for example, satellite, cellular, Wi-Fi, or BLUETOOTH technology. For example, network link 820 may provide a connection through a network 822 to a host computer 824.

Furthermore, network link 820 may provide a connection through network 822 or to other computing devices via internetworking devices and/or computers that are operated by an Internet Service Provider (ISP) 826. ISP 826 provides data communication services through a world-wide packet data communication network represented as internet 828. A server computer 830 may be coupled to internet 828. Server 830 broadly represents any computer, data center, virtual machine or virtual computing instance with or without a hypervisor, or computer executing a containerized program system such as DOCKER or KUBERNETES. Server 830 may represent an electronic digital service that is implemented using more than one computer or instance and that is accessed and used by transmitting web services requests, uniform resource locator (URL) strings with parameters in HTTP payloads, API calls, app services calls, or other service calls. Computer system 800 and server 830 may form elements of a distributed computing system that includes other computers, a processing cluster, server farm or other organization of computers that cooperate to perform tasks or execute applications or services. Server 830 may comprise one or more sets of instructions that are organized as modules, methods, objects, functions, routines, or calls. The instructions may be organized as one or more computer programs, operating system services, or application programs including mobile apps. The instructions may comprise an operating system and/or system software; one or more libraries to support multimedia, programming or other functions; data protocol instructions or stacks to implement TCP/IP, HTTP or other communication protocols; file format processing instructions to parse or render files coded using HTML, XML, JPEG, MPEG or PNG; user interface instructions to render or interpret commands for a graphical user interface (GUI), command-line interface or text user interface; application software such as an office suite, internet access applications, design and manufacturing applications, graphics applications, audio applications, software engineering applications, educational applications, games or miscellaneous applications. Server 830 may comprise a web application server that hosts a presentation layer, application layer and data storage layer such as a relational database system using structured query language (SQL) or no SQL, an object store, a graph database, a flat file system or other data storage.

Computer system 800 can send messages and receive data and instructions, including program code, through the network(s), network link 820 and communication interface 818. In the Internet example, a server 830 might transmit a requested code for an application program through Internet 828, ISP 826, local network 822 and communication interface 818. The received code may be executed by processor 804 as it is received, and/or stored in storage 810, or other non-volatile storage for later execution.

The execution of instructions as described in this section may implement a process in the form of an instance of a computer program that is being executed and consisting of program code and its current activity. Depending on the operating system (OS), a process may be made up of multiple threads of execution that execute instructions concurrently. In this context, a computer program is a passive collection of instructions, while a process may be the actual execution of those instructions. Several processes may be associated with the same program; for example, opening up several instances of the same program often means more than one process is being executed. Multitasking may be implemented to allow multiple processes to share processor 804. While each processor 804 or core of the processor executes a single task at a time, computer system 800 may be programmed to implement multitasking to allow each processor to switch between tasks that are being executed without having to wait for each task to finish. In an embodiment, switches may be performed when tasks perform input/output operations, when a task indicates that it can be switched, or on hardware interrupts. Time-sharing may be implemented to allow fast response for interactive user applications by rapidly performing context switches to provide the appearance of concurrent execution of multiple processes simultaneously. In an embodiment, for security and reliability, an operating system may prevent direct communication between independent processes, providing strictly mediated and controlled inter-process communication functionality.

In the foregoing specification, embodiments of the invention have been described with reference to numerous specific details that may vary from implementation to implementation. The specification and drawings are, accordingly, to be regarded in an illustrative rather than a restrictive sense. The sole and exclusive indicator of the scope of the invention, and what is intended by the applicants to be the scope of the invention, is the literal and equivalent scope of the set of claims that issue from this application, in the specific form in which such claims issue, including any subsequent correction.

Also discloses herein are the following numbered clauses:
1. A computer-implemented method executed using one or more computing devices and comprising:
   receiving, from a mobile computing device, a measurement-based care (MBC) assessment request from a patient;
   asynchronously assessing, using an application server computer, a plurality of MBC assessment data from the patient, wherein the plurality of MBC assessment data comprises passively collected data values, assessments, programmed heuristics, programmed rules, and model thresholds;
   asynchronously generating, using a machine learning model and the application server computer, a plurality of vital sign values for the patient using the plurality of MBC assessment data from the patient;
   asynchronously determining, using a machine learning model and the application server computer, stratification group data for the patient using the plurality of MBC assessment data from the patient; and
   sending, to the mobile computing device, instructions for presenting a user interface comprising the stratification group data for the patient.
2. The computer-implemented method of clause 1, further comprising launching a mobile application on the mobile computing device, wherein the mobile computing device can be a mobile computing device.
3. The computer-implemented method of clause 2, further comprising performing user authentication for the patient from the mobile computing device in an onboarding process for the patient, wherein the mobile computing device transmits an application protocol message to the application server computer or a third party service to causes transmitting an authentication code to a mobile phone number of the patient, to generate and cause displaying a prompt to receive the authentication code, to validate the authentication code, and to continue execution of the mobile application.
4. The computer-implemented method of clause 2 or 3, further comprising applying a two-factor authentication approach based on username and password to authenticate the patient.
5. The computer-implemented method of any preceding clause, further comprising generating the machine learning model to determine the stratification group data using a training dataset which is associated with inferred data values based on patient activity, semantic location, passive social determinants of health, self-reported information on mental health state, patient past diagnosis, patient demographic details, clinical action labels indicating what action a clinician took on a presenting patient taken from electronic health records (EHRs).
6. The computer-implemented method of any preceding clause, further comprising asynchronously determining, using an auto encoder and the application server computer, an attention level for the patient using the plurality of MBC assessment data from the patient.
7. The computer-implemented method of clause 6, wherein the auto encoder includes a contrastive loss function based on a first distance between anchor data and positive data, a second distance between the anchor data and negative data, and a margin.
8. The computer-implemented method of any preceding clause, further comprising:
   generating a trend report for each of the plurality of vital sign values using the plurality of MBC assessment data from the patient, wherein the trend report includes, for each of the plurality of vital sign values, a respective current state over a period of time and a respective valence value, and a respective trend; and
   determining severity associated with the plurality of vital sign values based on the trend report.
9. The computer-implemented method of any preceding clause, further comprising:
   ranking the patient using the patient's attention level; and
   assigning a category to the patient using the patient's attention level.
10. The computer-implemented method of clause 9, wherein the category is one selected from the group consisting of absence of concerning signals, take note/lower risk anomalies, urgent/higher risk anomalies, and emergency/in need of attention.
11. The computer-implemented method of any preceding clause, further comprising performing random clinical assessments for the patient, wherein the random clinical assessments for the patient are triggered by passive data anomaly detection, clinical heuristics, and clinician.
12. A distributed computer system comprising:
   a mobile computing device;
   an application server computer;
   one or more processors; and
   one or more computer-readable non-transitory storage media coupled to one or more of the processors and comprising instructions operable when executed by one or more of the processors to cause the one or more processors to perform the operations recited in any of clause 1 to clause 11.
13. One or more computer-readable non-transitory storage media storing one or more sequences of instructions which when executed by one or more processors cause the one or more processors to perform the operations recited in any of clause 1 to clause 11.

## Claims

1. A computer-implemented method executed using one or more computing devices and comprising:
receiving, from a mobile computing device, a measurement-based care (MBC) assessment request from a patient;
asynchronously assessing, using an application server computer, a plurality of MBC assessment data from the patient, wherein the plurality of MBC assessment data comprises passively collected data values, assessments, programmed heuristics, programmed rules, and model thresholds;
asynchronously generating, using a machine learning model and the application server computer, a plurality of vital sign values for the patient using the plurality of MBC assessment data from the patient;
asynchronously determining, using a machine learning model and the application server computer, stratification group data for the patient using the plurality of MBC assessment data from the patient; and
sending, to the mobile computing device, instructions for presenting a user interface comprising the stratification group data for the patient.

2. The computer-implemented method of Claim 1, further comprising launching a mobile application on the mobile computing device, wherein the mobile computing device can be a mobile computing device.

3. The computer-implemented method of Claim 2, further comprising performing user authentication for the patient from the mobile computing device in an onboarding process for the patient, wherein the mobile computing device transmits an application protocol message to the application server computer or a third party service to causes transmitting an authentication code to a mobile phone number of the patient, to generate and cause displaying a prompt to receive the authentication code, to validate the authentication code, and to continue execution of the mobile application.

4. The computer-implemented method of Claim 2, further comprising applying a two-factor authentication approach based on username and password to authenticate the patient.

5. The computer-implemented method of any preceding Claim, further comprising generating the machine learning model to determine the stratification group data using a training dataset which is associated with inferred data values based on patient activity, semantic location, passive social determinants of health, self-reported information on mental health state, patient past diagnosis, patient demographic details, clinical action labels indicating what action a clinician took on a presenting patient taken from electronic health records (EHRs).

6. The computer-implemented method of any preceding Claim, further comprising asynchronously determining, using an auto encoder and the application server computer, an attention level for the patient using the plurality of MBC assessment data from the patient.

7. The computer-implemented method of Claim 6, wherein the auto encoder includes a contrastive loss function based on a first distance between anchor data and positive data, a second distance between the anchor data and negative data, and a margin.

8. The computer-implemented method of any preceding Claim, further comprising:
generating a trend report for each of the plurality of vital sign values using the plurality of MBC assessment data from the patient, wherein the trend report includes, for each of the plurality of vital sign values, a respective current state over a period of time and a respective valence value, and a respective trend; and
determining severity associated with the plurality of vital sign values based on the trend report.

9. The computer-implemented method of any preceding Claim, further comprising:
ranking the patient using the patient's attention level; and
assigning a category to the patient using the patient's attention level.

10. The computer-implemented method of Claim 9, wherein the category is one selected from the group consisting of absence of concerning signals, take note/lower risk anomalies, urgent/higher risk anomalies, and emergency/in need of attention.

11. The computer-implemented method of any preceding Claim, further comprising performing random clinical assessments for the patient, wherein the random clinical assessments for the patient are triggered by passive data anomaly detection, clinical heuristics, and clinician.

12. A distributed computer system comprising:
a mobile computing device;
an application server computer;
one or more processors; and
one or more computer-readable non-transitory storage media coupled to one or more of the processors and comprising instructions operable when executed by one or more of the processors to cause the one or more processors to perform the operations recited in any of claim 1 to claim 11.

13. One or more computer-readable non-transitory storage media storing one or more sequences of instructions which when executed by one or more processors cause the one or more processors to perform the operations recited in any of claim 1 to claim 11.
